# EUROPEAN PATENT APPLICATION

(11) **EP 3 816 280 A1**
(43) Date of publication of application: **05.05.2021**
(21) Application number: 19206580.3
(22) Date of filing: 31.10.2019
(51) Int. Cl.: C12N 7/00, A61K 35/76, A61P 31/04

(54) **COLIPHAGES AND USES THEREOF**

(71) Applicant: Institut national de recherche pour l'agriculture, l'alimentation et l'environnement, 75007 Paris (FR); Gentofte Hospital, 2900 Hellerup (DK); University of Copenhagen, 1165 Copenhagen K (DK); Université Laval, Québec, Québec G1V 0A6 (CA)
(72) Inventor: PETIT, Marie-Agnès, 75010 Paris (FR); MATHIEU, Aurélie, 92120 Montrouge (FR); BISGAARD, Hans, 2930 Klampenborg (DK); SHAH, Shiraz, 2860 Søborg (DK); NIELSEN, Dennis Sandris, 2000 Frediksberg (DK); DENG, Ling, 3500 Hareskov (DK); MOINEAU, Sylvain, Quebec, G1W2S5 (CA); DION, Moïra, Quebec, G1C7M4 (CA)
(74) Representative: Regimbeau

(57) **Abstract**

The present invention relates to bacteriophages and their use in therapy. More particularly, the present invention relates to novel bacteriophages having a lytic against *Escherichia coli* strains, their manufacture, components thereof, compositions comprising the same and the uses thereof in phage therapy.

## Description

### INTRODUCTION

The present invention relates to bacteriophages and their use in therapy. More particularly, the present invention relates to novel bacteriophages having a lytic activity against *Escherichia coli* strains, their manufacture, components thereof, compositions comprising the same and the uses thereof in phage therapy.

Bacteriophages (or phages), literally *"eaters of bacteria",* are small viruses displaying the ability to infect and kill bacteria while they do not affect cells from other organisms. Initially described almost a century ago by William Twort, and independently discovered shortly thereafter by Félix d'Herelle, more than 6000 different bacteriophages have been discovered and described morphologically, including bacterial and archeal viruses. The vast majority of these viruses are tailed while a small proportion are polyhedral, filamentous or pleomorphic. They may be classified according to their morphology, their genetic content (DNA vs. RNA), their specific host, the place where they live (marine virus vs. other habitats), and their life cycle. As intra-cellular parasites of bacterial cells, phages display different life cycles within the bacterial host: lytic, lysogenic, pseudo-lysogenic, and chronic infection (Weinbauer MG, FEMS Microbiol Rev, 2004, 28:127-81; Drulis-Kawa et al., Curr Protein Pept Sci, 2012, 13(8):699-722). Depending on which cycles they can perform, phages are divided into two main categories: virulents and temperates. Virulent phages only perform lytic cycles and therefore cause lysis of the host bacterium. Temperate phages alternate between lytic life cycles, whereby they cause lysis of the host bacterium, and lysogenic cycles, whereby they incorporate their DNA into the host bacterial DNA and become prophages. Irrespective of the phage category, the first step is the attachment to receptors of the bacterial cell wall before phages may enter the bacteria. This specific process influences the spectrum of the possible phage-bacteria interactions.

Because of their target host cell specificity, the use of phages as a therapy to treat infections, whether chronic or acute, has been considered, particularly in dermatology, ophthalmology, urology, stomatology, pediatrics, otolaryngology or surgery. This concept of therapeutic use of phages to treat bacterial infection was, however, highly controversial from the very beginning and not widely accepted by the public or medical community. Early studies were widely criticized for lack of appropriate controls and inconsistent results. The lack of reproducibility and many conflicting results obtained in the various published studies led the Council on Pharmacy and Chemistry of the American Medical Association to conclude that the evidence for the therapeutic value of lytic filtrates was for the most part contradictory, unconvincing, and recommended additional research to confirm its purported benefits.

Since the introduction of antibiotics in the 1940s, little attention was paid to this field of therapeutics, especially in the Western world. But the extensive use of antibiotics has led to the widespread emergence and spread of antibiotic-resistant bacteria around the world, causing increasingly serious problems. It has therefore become a major therapeutic challenge to overcome the limited therapeutic options remaining to treat major multi-drug resistant microbes.

*E. coli,* a Gram-negative, short rod-shaped bacterium belonging to the genus Escherichia and the family Enterobacteriaceae, shows high diversity and frequency in human or animal microbial flora. The *E. coli* species is diverse, and its phylogeny is particularly well studied. Seven phylogroups are distinguished, with different overall properties. Clades A and B2 are the most prevalent in humans, with A strains being mostly commensals while most extra-intestinal pathogens belong to the B2 clade. It was revealed that while most strains of *E. coli* are non-pathogenic, they can cause opportunistic infections. Furthermore, some *E. coli* strains are highly pathogenic and can cause diverse diseases and sepsis in mammals, including humans. Several reports associate the enterobacterium *Escherichia coli* with skin and soft tissue infections (SSTIs), one of the most common infections in patients of all age groups. In some moderate or severe cases, these infections require hospitalization and parenteral therapy. *E. coli* was notably found to be the causative agent of neonatal omphalitis (Fraser and al, 2006), cellulitis localized to lower or upper limbs (Brzozowski and al, 1997, Corredoira and al, 1994), necrotizing fasciitis (Afifi and al, 2008; Krebs and al, 2001), surgical site infections (Tourmousoglou and al, 2008), infections after burn injuries (Rodgers and al, 2000), and others. A study monitoring SSTIs during a 7-year period and encompassing three continents (Europe, Latin America, and North America) showed *E. coli* to be an important causative agent, since it was the third-most prevalent isolated species. *E. coli* therefore deserves specific and targeting therapy, especially taking into account the dramatic decline in antibiotic susceptibility of pathogenic *E. coli* strains in recent years, their diversity, and their substantial presence in microbial flora.

Furthermore, it has been reported that the pathological or physiological condition of a subject is influenced by the balance of microorganisms in the flora of the subject. Accordingly, modifying the microbial flora, or modifying said balance, or restoring said balance, by destroying *E. coli* population, is also a valuable approach for improving a subject's condition.

There is therefore a great need for new antibacterial agents or compositions that can be used to destroy *E. coli* strains, even when organized in bacterial biofilms, suitable for use in human or animal therapy as well as for decontaminating products.

*E. coli* being one of the first and most prevalent bacterial species to colonize an infant's gut, the Inventors have herein investigated for the first time the presence of coliphages in infants' gut virome. They successfully isolated and characterized new bacteriophages belonging to the virulent category and killing a wide range of *Escherichia coli* (*E*. *coli*) strains, and which can be used as active agents in pharmaceutical or veterinary preparations, particularly to treat *E. coli* bacterial infections or to modify microbial balance in a subject. These new bacteriophages of the invention exhibit strong lytic activity, and can by combined to induce controlled destruction of a very large spectrum of *E. coli* strains.

### SUMMARY OF THE INVENTION

In a first aspect, the invention relates to a bacteriophage having lytic activity to an *Escherichia coli* strain, said bacteriophage having a genome comprising or consisting of a nucleotide sequence selected from any one of SEQ ID NO: 1 to 15 or a nucleotide sequence having at least 97% identity thereto.

In another aspect, the invention relates to a nucleic acid isolated from a bacteriophage according to the invention.

Yet, in another aspect, the invention relates to a polypeptide isolated from a bacteriophage according to the invention.

In another aspect, the invention relates to an antibacterial composition comprising at least one bacteriophage according to the invention. In particular, the antibacterial composition comprises at least two, at least three, preferably at least four distinct bacteriophages having lytic activity to an *Escherichia coli* strain, said bacteriophages being selected from the bacteriophages having a genome comprising or consisting of a nucleotide sequence selected from any one of SEQ ID NO: 1 to 15 or a sequence having at least 90% identity thereto.

In a further aspect, the invention is directed to a bacteriophage, a nucleic acid, a polypeptide, or an antibacterial composition as described herein, for use as a medicament, preferably for treating an *E. coli* infection, or for restoring the microbial flora in a subject. In other words, the invention provides the use of said bacteriophage, nucleic acid, polypeptide, or antibacterial composition, for the preparation of a medicament, in particular for treating an *E. coli* infection or for restoring the microbial flora in a subject. More precisely, the invention provides a method for treating a subject in need thereof, such as a subject suffering an *E. coli* infection or in need of restoration of microbial flora, comprising the administration of a therapeutically effective amount of said bacteriophage, nucleic acid, polypeptide, or antibacterial composition to said subject.

Another aspect of the invention is to provide an *in vitro* use of a bacteriophage, a nucleic acid, a polypeptide, or an antibacterial composition as disclosed herein, for decontaminating a product contaminated with *E. coli.*

Still, a further aspect of the invention resides in at least two distinct bacteriophages, at least two distinct nucleic acids, or at least two distinct polypeptides as disclosed herein, as a combined preparation for simultaneous, separate or sequential use in therapy, such as in a treatment of an *E. coli* infection or for restoring the microbial flora in a subject.

In another aspect, the invention pertains to an *in vitro* use of at least two distinct bacteriophages, at least two distinct nucleic acids, or at least two distinct polypeptides as disclosed herein, as a combined preparation for simultaneous, separate or sequential decontamination of a product contaminated with *E. coli.*

Yet, in another aspect, the invention provides a method for predicting or determining the efficacy of a bacteriophage therapy in a subject, comprising determining *in vitro* a lytic activity of one or more bacteriophage, nucleic acid, polypeptide, or antibacterial composition as described herein, to an *E. coli* strain from a sample of said subject, wherein the presence of a lytic activity of said bacteriophage, nucleic acid, polypeptide, or antibacterial composition, to said *E. coli* strain from said sample is indicative of an efficient treatment.

In a last aspect, the invention pertains to a method for selecting a subject or determining whether a subject is susceptible to benefit from a bacteriophage therapy, comprising determining *in vitro* a lytic activity of one or more bacteriophage, nucleic acid, polypeptide, or antibacterial composition as described herein, to an *E. coli* strain from a sample of said subject, wherein the presence of a lytic activity of said bacteriophage, nucleic acid, polypeptide, or antibacterial composition, to said *E. coli* strain from said sample is indicative of a responder subject.

### LEGENDS TO THE FIGURES

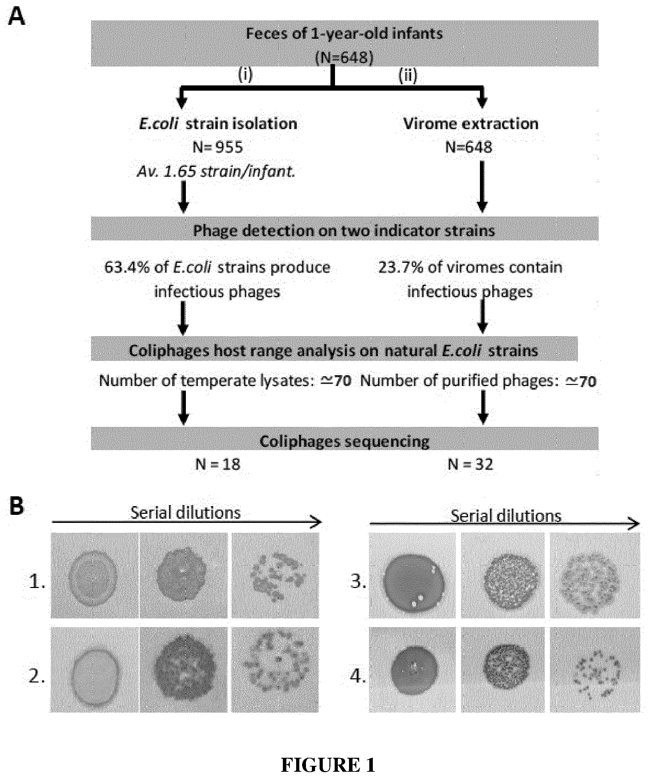

**Figure 1****. Coliphages from 1-year-old infant fecal microbiota: overview of the study**
   **(A)** Fecal samples from 649 1-year-old infants were collected and used (i) to isolate *E. coli* strains and their resident temperate phages and (ii) to extract their viromes. Next, two laboratory strains were chosen to culture coliphages from these two sources. Subsequently, using a subset of roughly 70 coliphages from each source, the host-range was determined based on at least 70 *E. coli* isolates from the same cohort. Finally, the genomes of 18 temperate coliphages isolated from *E. coli* strains and 32 coliphages isolated in viromes were sequenced. **(B)** Examples of temperate/virulent phenotypes of the coliphages isolated from viromes. Various dilutions of coliphages amplified on either of the two indicator strains were spotted on soft agar plates inoculated with an *E. coli* indicator strain. Confluent growth and isolated plaques of two phages making turbid (1 and 2) and clear (3 and 4) plaques are shown.

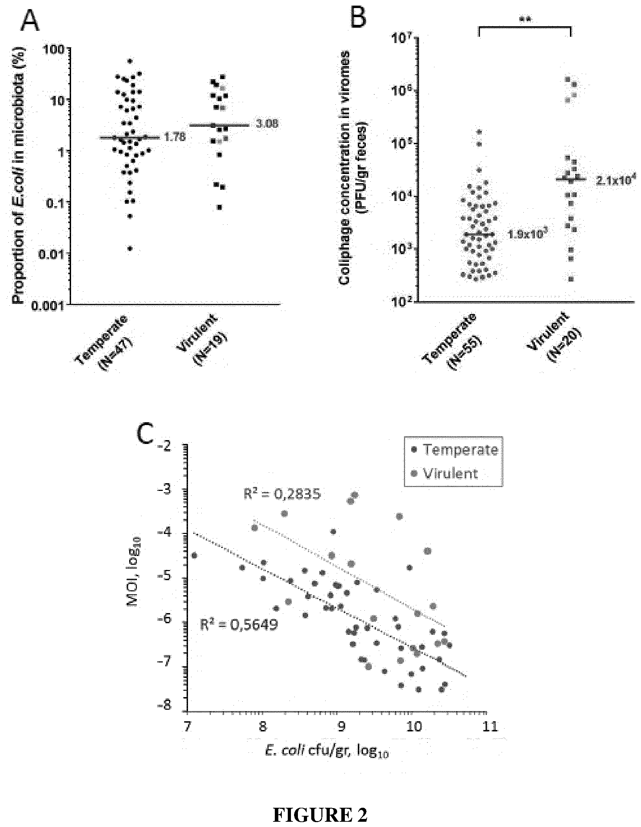

**Figure 2****. *E. coli* and coliphage levels in microbiota segregated by lifestyle. (A)** A slight increase of the fraction *of E. coli* to total microbiota OTU is found among samples containing virulent coliphages, compared to those where temperate phages were found (not statistically significant, Mann-Whitney test, P=0.39). **(B)** The overall coliphages titer in the fecal sample is significantly higher for virulent, compared to temperate phages (**, P value = 0.0002). In both first panels, straight line = median value. **(C)** The multiplicity of infection (MOI) correlates negatively with *E. coli* concentration in the fecal samples, both for virulent and temperate phage containing samples (R2, coefficient of determination of the power law).

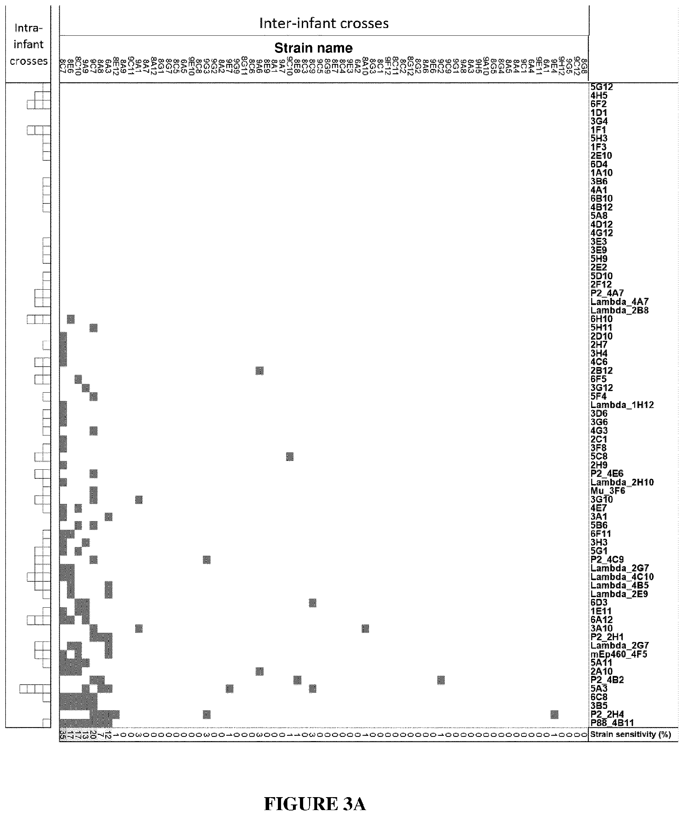

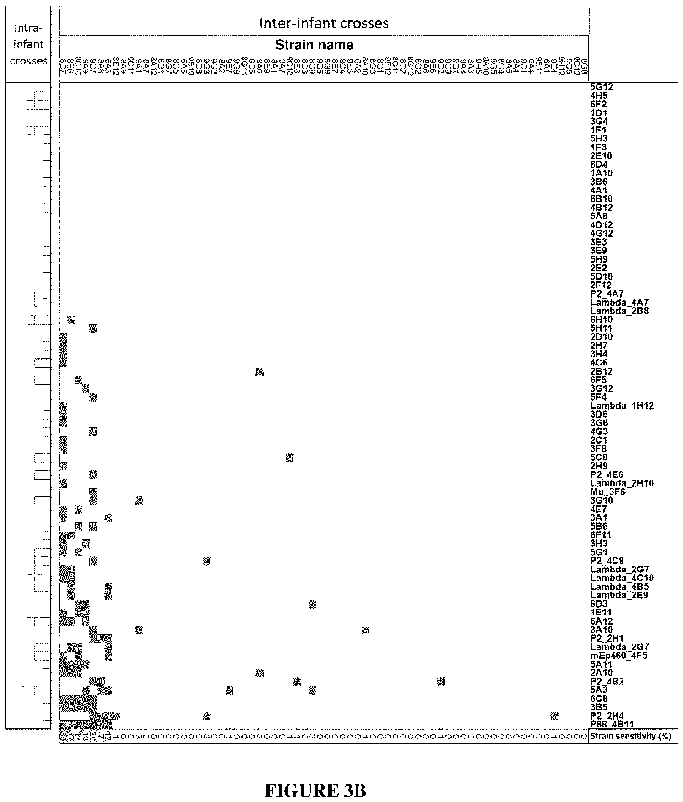

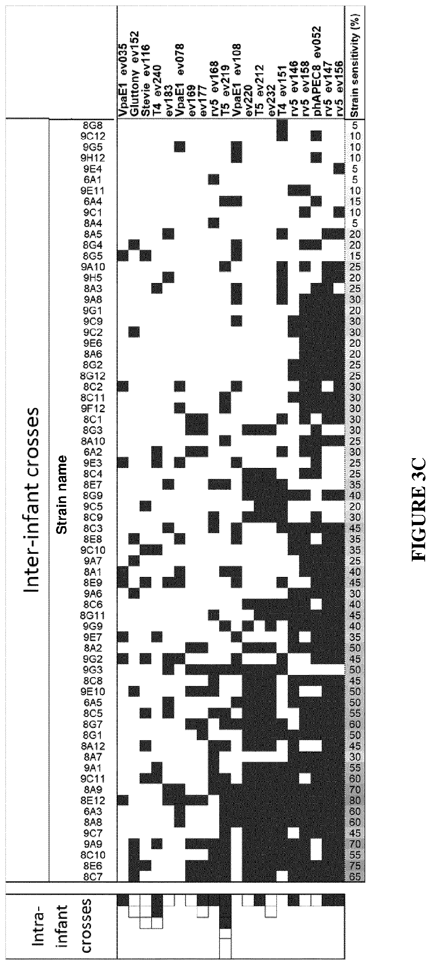

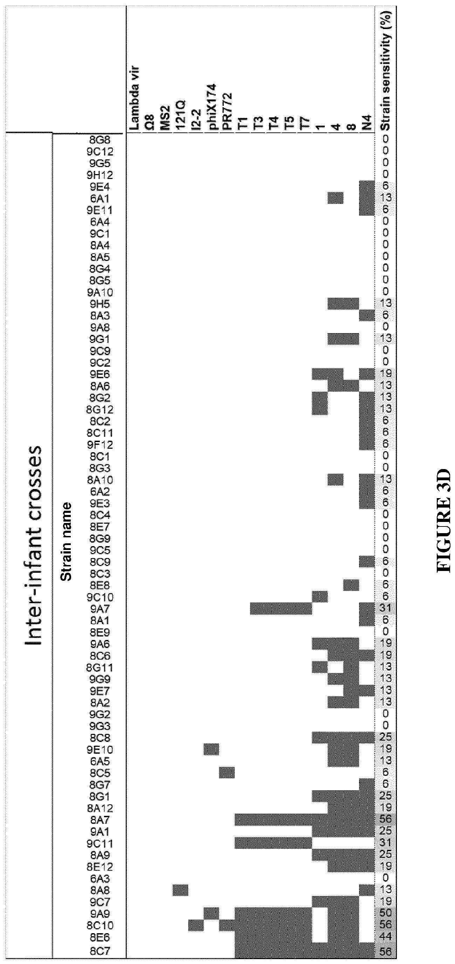

**Figure 3****. Phage impact on natural *E. coli* strains.** Bacterial lawns *of E. coli* isolates from infant fecal samples were infected with hundreds of coliphages. The upper row shows each *E*. *coli* strain name, which belongs to 7 main phylo-groups (A, B1, B2, C, D, E and E-clade). The left column indicates each phage name, which are categorized into 3 main groups: temperate phages from natural *E. coli* strain (A), temperate phages isolated from viromes (B) and virulent phages isolated from viromes (C). Each square of the matrix corresponds to a phage-strain interaction (empty square: no phage growth, filled square: phage growth). In panels (A) and (B), all crosses are inter-infant (i.e. phage and strain never come from the same infant). Intra-infant crosses (infant coliphages tested on the strains originating from the same fecal sample) are shown on the left column. The *"strain sensitivity"* row sums up the percentage of phages in each category infecting this strain. **(A)** Temperate phages derived from fecal *E. coli* strains supernatants, either as crude lysates (black entry) or as plaque purified and amplified on an indicator strain. The *E. coli* strain from which the phage was collected was excluded. **(B)** Temperate coliphages found in viromes, plaque purified and amplified on an indicator strain. **(C)** Virulent coliphages found in viromes, plaque purified and amplified on an indicator strain **(D)** Reference virulent coliphages obtained from the Felix d'Hérelle Reference Center for Bacterial Viruses (www.phage.ulaval.ca).

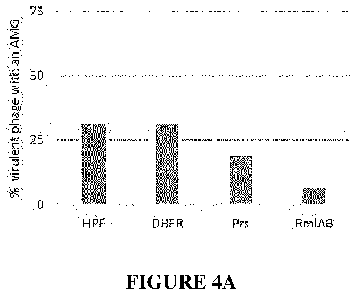

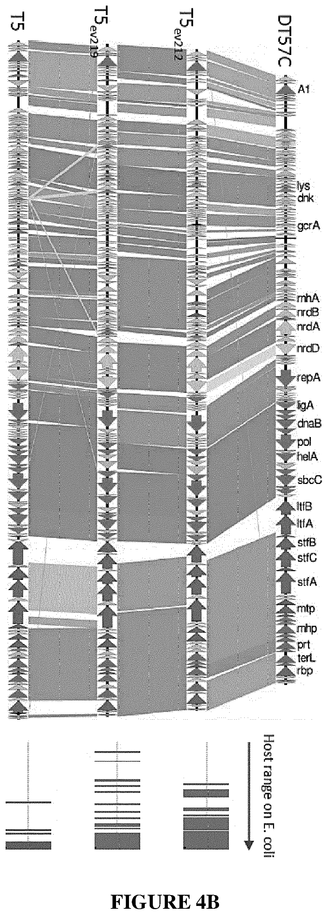

**Figure 4****. Salient features of virulent coliphage genomes. (A)** Auxiliary Metabolic Genes frequencies among 15 virulent sequenced phages. HPS, hibernation ribosomal protein; DHFR, dihydrofolate reductase; Prs, ribose-phosphate pyrophosphokinase; RmlA, glucose-1-phosphate thymidylyltransferase; RmlB, dTDP-glucose 4,6 dehydratase. **(B)** BLASTn alignment of four T5 genomes. The two T5-like phages isolated from viromes differ from T5 in their receptor binding protein (p05, similar to the DT57C gene), and from both DT57C and T5 in their L-shape tail fibers. Bottom panel: grey bars stand for strains on which each T5 grows.

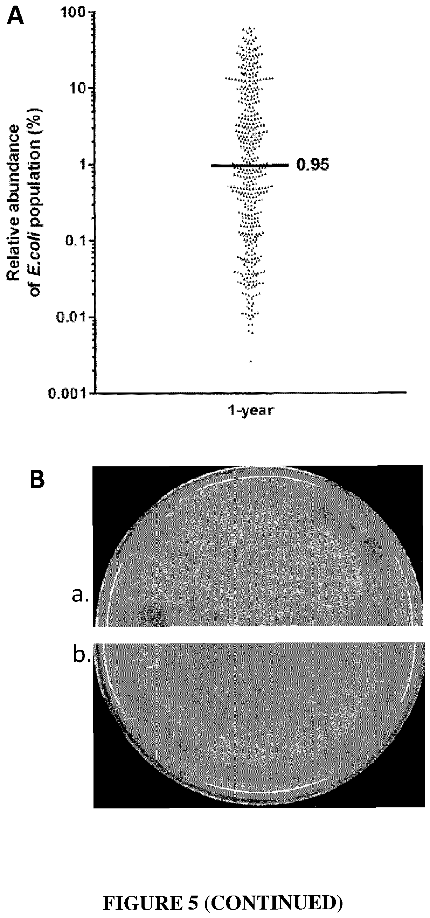

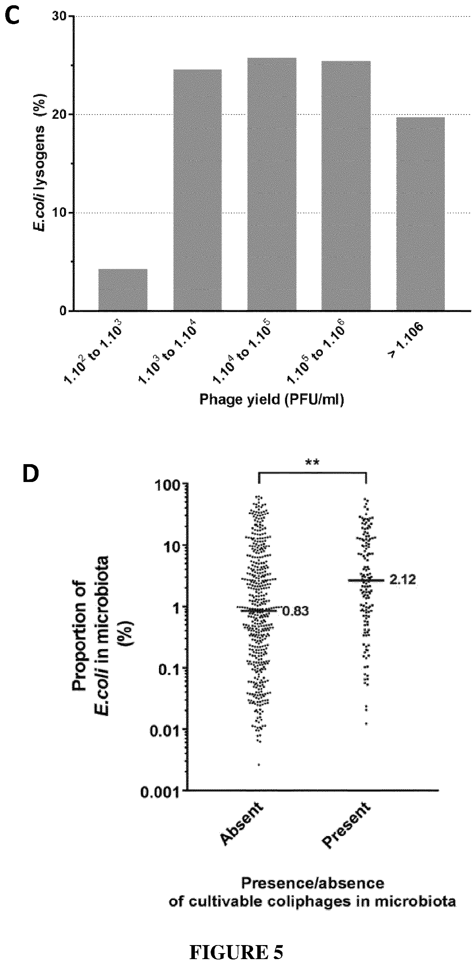

**Figure 5****. *E. coli* populations in fecal samples of 1-year-old infants. (A)** Relative abundance *of E. coli* populations in fecal samples from 1-year-olds. The straight line near the 0.95 value corresponds to the median value. **(B)** Lysis plaque assays. The supernatant of overnight (O/N) cultures of two *E. coli* strains were filtered and 10 µl were spotted on plate before adding the soft agar containing *E. coli* MAC1403 strain. Lysis plaques were observed after an O/N incubation period. (C) Distribution of phage yields in *E. coli* lysogen supernatants (PFU/ml). **(D)** The fraction of *E. coli* to total microbiota OTU is significantly higher among fecal samples from which cultivable coliphages were obtained. ** Mann-Whitney two-660 tailed test, P value < 0.0001.

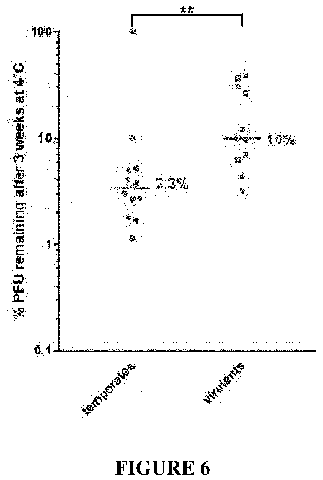

**Figure 6****. Coliphage stability at 4°C.** Lysates of 11 temperate and 12 virulent coliphages stored at 4°C were titrated at day 0 and 35 after phage production. Each point represents the average of the decay (% of initial titer) on two independent stocks. The difference in stability between temperate and virulent was significant (**, Mann-Whitney two-tailed test, P= 0.0059).

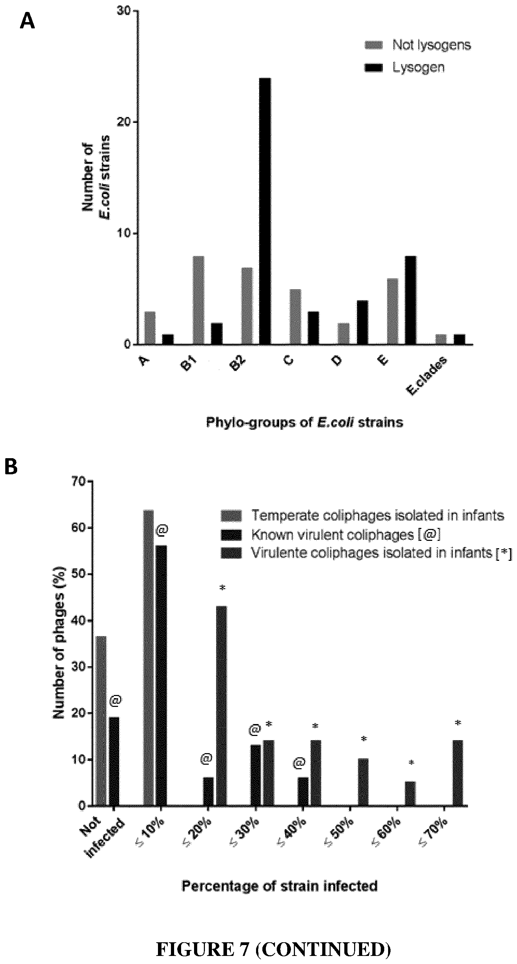

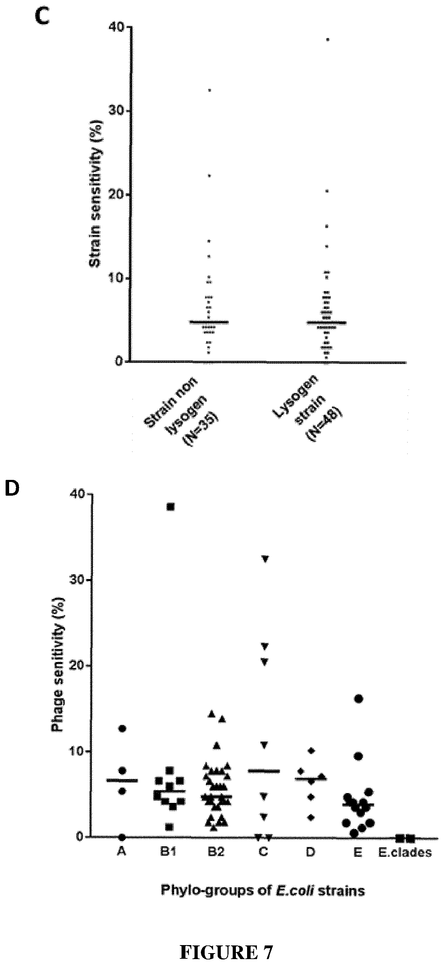

**Figure 7****. Phage-host interactions and phylogroups. (A)** Number of *E. coli* lysogens as a function of strain phylogroup. B2 had significantly more *E. coli* lysogens than the average (74 %), while A and B1 had significantly less *E. coli* lysogens than the average. **(B)** Three distinct profiles of coliphage infectivity on the natural *E. coli* strains according to the phage life-style (temperate or virulent). **(C)** Phage-sensitivity of the tested *E. coli* strains according to their phylo-groups. Results are not significantly different between phylo-groups. **(D)** Sensitivity *of E. coli* strains depending on whether or not strains are lysogens. Straight lines: median values.

### DETAILED DESCRIPTION OF THE INVENTION

The present invention relates to novel bacteriophages, components thereof, compositions comprising the same, their manufacture, components thereof, composition comprising the same, and the uses thereof as antibacterial agents, such as for the treatment of an infection in a subject in need thereof.

### Definitions

Unless otherwise defined herein, scientific and technical terms used in connection with the present invention shall have the meanings that are commonly understood by those of ordinary skill in the art. Further, unless otherwise required by context, nomenclatures used herein, and techniques of phage preparation are those well-known and commonly used in the art.

Nevertheless, with respect to the use of different terms throughout the current specification, the following definitions more particularly apply.

As used herein, the term *"bacteriophage"* or *"phage"* refers to a virus that can infect one or more types of bacterial strains. Phages are typically composed of proteins that encapsulate a DNA or RNA genome, which may encode a few or hundreds of genes, and that can produce functional virions (phage particles) i.e. progeny of the phage capable of further infecting neighboring bacteria. The term *"bacteriophage"* can thus refer herein to a functional phage particle comprising a nucleic acid genome packaged in a proteinaceous envelope or capsid. This term also encompasses portions of the bacteriophage, including, e.g., a head portion, or an assembly of phage components, which provide substantially the same functional activity as a whole phage particle.

It should be further understood that the bacteriophage according to the invention is preferably an *"isolated bacteriophage",* i.e. a bacteriophage removed from its native environment (e.g. herein the human gut) in which it is naturally present. As such, this term can designate for example a phage that is e.g. cultivated, purified and/or cultured separately from the environment in which it is naturally located.

A bacteriophage can be characterized according to its phenotype. The term *"phenotype"* or *"phenotypic characteristic(s)"* designates herein the structure and/or function and/or host-range of a bacteriophage.

With regard to their structure, phages are conventionally classified according to the International Committee on Taxonomy of Viruses (ICTV) based on their morphology and type of nucleic acid (DNA or RNA, single- or double-stranded, linear or circular). To this day, the double-stranded DNA tailed phages, belonging to the *Caudovirales* order, account for about 95% of all the phages reported in the literature, though there are also other phages that occur abundantly in the biosphere with different types of virions, genomes and lifestyles. So far, about 13 bacteriophage families have been recognized among the *Caudovirales* order as reported in Table 1 below.

**Table 1. ICTV classification of Caudovirales phages (adapted from McAufliffe et al., Bacteriophage: Genetics and Molecular Biology by Stephen Mc Grath et al., 2007, Caister Academic Press)**

| **Family** | **Morphology** | **Nucleic acid** |
|---|---|---|
| *Myoviridae* | Non-enveloped, contractile tail | Linear dsDNA |
| *Siphoviridae* | Non-enveloped, long contractile tail | Linear dsDNA |
| *Podoviridae* | Non-enveloped, long non-contractile tail | Linear dsDNA |
| *Tectiviridae* | Non-enveloped, short non-contractile tail | Linear dsDNA |
| *Cortoviridae* | Non-enveloped, isometric | Circular dsDNA |
| *Lipothrixviridae* | Enveloped, rod-shaped | Linear dsDNA |
| *Plasmaviridae* | Enveloped, pleomorphic | Circular dsDNA |
| *Rudiviridae* | Non-enveloped, rod-shaped | Linear dsDNA |
| *Fuselloviridae* | Non-enveloped, lemon-shaped | Circular dsDNA |
| *Inoviridae* | Non-enveloped, filamentous | Circular ssDNA |
| *Microviridae* | Non-enveloped, isometric | Circular ssDNA |
| *Leviviridae* | Non-enveloped, isometric | Linear ssRNA |
| *Cystoviridae* | Enveloped, spherical | Segmented dsRNA |

Bacteriophages identified by the present Inventors are either myophages (they belong to the *Myoviridae* family) or siphophages (they belong to the *Siphoviridae* family). Because the ICTV classification could lead to separate phages having closely related genomes, a more detailed classification of viruses has been developed in the recent years based on homology search of neck proteins of known phages (Lopes A et al., Nucleic Acids Res., 2010, 38(12):3952-62; Lopes A et al., BMC Genomics, 2014, 15:1027). Upon identification of a new phage genome, distant homology search can be automatically performed using the VIRFAM platform (Remote Homology Detection of Viral Protein Families, CEA website http://biodev.cea.fr/virfam/), for each set of proteins identified in said phage. As of today, the VIRFAM classification is divided into 5 types, named "*1*", "*2*", "*3*", "*4*" and *"unassigned",* based on the types of phage neck identified by homology. The neck represents the head-to-tail interface containing at least an adaptor protein (Ad), but also up to three other components named Hc (head closure protein), Tc (tail completion protein), and Ne (neck protein).

**Table 2. VIRFAM classification of tailed bacteriophages (Ad: Adaptor protein, Hc: Head-closure protein, Ne: Neck protein, Tc: Tail-completion protein)**

| **VIRFAM Neck Type** | Neck proteins | **VIRFAM Cluster** |
|---|---|---|
| | | Phage representative of neck structure (bacterial host) |
| **1** | Ad1, Hc1, Ne1, Tc1 | 1: SPP1 (Firmicutes) |
| | | 2: phi-105 (Firmicutes) |
| | | 3: HK97 (β and γ proteobacteria) |
| | | 4: Rosebush (Actinobacteria) |
| | | 5: PA73 (β and γ proteobacteria) |
| | | 6: lambda-like (β and γ proteobacteria) |
| | | 7: Aaphi23 (β and γ proteobacteria) |
| | | 8: Mu (β and γ proteobacteria) |
| | | 9: RSA1 (β and γ proteobacteria) |
| | | 10: Bxb1 (Actinobacteria/Firmicutes) |
| **2** | Ad2, Hc2, Tc2 | T4 |
| **3** | Ad3, Hc3 | P22 |
| **4** | Ad4 | Phi29 |
| **unassigned** | - | - |

As explained above, phages can be broadly divided into two functional categories: *"virulent phages"* which perform only lytic cycles, and *"temperate phages"* that switch between lysogenic and lytic cycles. Upon infection of the bacterial host, virulent phages immediately commandeer the bacterial replicative machinery in order to multiply, while temperate phages follow either one of two routes, depending on the physiology of its host: lytic cycle or lysogenic cycle, an alternative life cycle involving the integration of their genome into the host chromosome to become a prophage. The prophage will eventually switch to a lytic life cycle again, in response to environmental stresses. The bacteriophages according to the present invention are herein virulent phages; as such, their activity is strictly lytic as defined below, as they do not undergo a lysogenic stage.

Bacteriophages can also be defined according to their host-range, i.e. the breadth of bacterial genera, species or strains it is capable of infecting. Such phenotypic characteristic can be directly assessed *in vitro* by inoculating different bacterial genera, species or strains with a phage, via for example a spot assay or a plaque assay, or predicted via computational approaches (Edwards et al., FEMS Microbiol Rev., 2016, 40(2):258-72). As explained below, the bacteriophages according to the present invention are capable of infecting the bacterial species *Escherichia coli* and can thus be referred as coliphages. Depending on its host range, a bacteriophage can be defined as being specific to one or more bacterial genera, species or strains, meaning that it can infect said bacterial genus, species or strains but not other ones. For example, a bacteriophage *"specific"* for *E. coli* typically designates a bacteriophage which can infect one or more distinct *E. coli* strains and which cannot infect non-*E*. *coli* bacteria. Specificity is usually mediated by the tail fibers of bacteriophages, since these fibers are involved in the interaction with receptors expressed on host cells.

The term *"lytic activity"* refers herein to the property of a bacteriophage to cause lysis of a bacterial cell. A lytic bacteriophage is one that follows the lytic pathway through completion of the lytic cycle, rather than entering the lysogenic pathway. A lytic bacteriophage typically undergoes viral replication in the host bacterial cell it has infected, leading to lysis of the bacterial cell membrane, destruction of the cell, and release of progeny bacteriophage particles capable of infecting other bacterial cells. In the context of the present invention, the bacteriophage is lytic against *E. coli.* The lytic activity of the bacteriophage can thus be assessed *in vitro* by infecting one or more *E. coli* strains of interest with said phage using techniques well-known in the art, such as those described in details in the Example below. This lytic activity can be more particularly assessed by measuring the PLE and/or PFU of a bacteriophage on the selected *E. coli* strain.

The term "*PLE*" or *"Productive Lytic Effect"* designates the ratio between burst size and productive lytic time of a given bacteriophage. Burst size and productive lytic time are parameters defining phage-host interaction and correspond, respectively, to the mean yield of bacteriophage particles produced by infection of one bacterium by one phage, and to the time taken by a free bacteriophage to lyse a bacterial cell.

As used herein, "*PFU*" means plaque forming unit. It is a measure of number of infectious virus particles, and is typically determined by a plaque forming assay. A plaque assay, also known as double agar method, is based on the growing of bacteriophage with potential host bacteria followed by the assessment of the ability of said phage to kill the bacterial cells. Typically, during such assay, lytic bacteriophages lyse the host cell after a period of incubation in soft agar medium, thereby causing a zone of clearing (or plaque) on a culture plate. Theoretically, each plaque is formed by one phage and the number of plaques multiplied by the dilution factor is equal to the total number of phages in a test preparation.

The term "*MOI*" means multiplicity of infection, as well-known in the art of virology. It is the average number of virus particles infecting each cell. MOI is related to PFU by the following formula: MOI = PFU of a given virus used for infection / number of cells. For example, if 2x10⁶ cells are infected by 50 µl of virus with a titer of 10⁸ pfu/ml, then the MOI will be 0.05^{∗}10⁸/2^{∗}10⁶ = 2.5.

The present invention also embraces variants of the bacteriophages disclosed herein. By *"variant"* of a given bacteriophage (reference bacteriophage), it is meant herein a phage having variation(s) in the genomic sequence and/or polypeptide(s) encoded thereby as compared to said reference bacteriophage, and retaining the same or substantially the same phenotypic characteristic(s) as the reference bacteriophage. Variants typically comprise e.g. silent mutations, conservative mutations, minor deletions, and/or minor replications of genetic material, which do not impact or substantially impact the phenotypic characteristic(s) of the reference bacteriophage. In a preferred embodiment, the variant of the invention retains any observable characteristic or property that is dependent upon the genome of the bacteriophage of the invention, e.g. genes of said bacteriophage which are responsible for its lytic activity towards *E. coli.* Preferred variants have less than 5% nucleic acid variation as compared to the genome of the reference bacteriophage, even more preferably less than 4%, more preferably less than 2%. In other words, preferred variants have more than 95% nucleic acid sequence identity as compared to the genome of reference bacteriophage, even more preferably more than 96%, more preferably more than 98% sequence identity. Alternatively, or in combination, variants have preferably less than 5% amino acid variation in a coded polypeptide sequence as compared to a polypeptide of the reference bacteriophage. That is, variants have preferably more than 95% amino acid sequence identity in a coded polypeptide sequence as compared to a polypeptide of the reference bacteriophage.

By *"nucleotide", "nucleic acid", "nucleotide sequence"* or *"nucleic acid sequence",* it is meant herein a precise succession of natural nucleotides (namely, A, T, G, C and U) or non-natural nucleotides, corresponding to a single-stranded (ss) or double-stranded (ds) DNA such as genomic DNA, or to the transcription product of said DNA, such as an RNA. Said nucleic acid may further be linear or circular. In the context of the present invention, the nucleic acid is preferably in the form of DNA, more preferably double-stranded DNA, even more preferably linear double-stranded DNA.

As used herein, the terms *"polypeptide", "protein"* and *"peptide"* are used interchangeably to refer to a precise succession of amino acid residues. As such, these terms include polypeptides of any size, including small peptides of e.g., from 5 to 20 amino acids or longer polypeptides, as well as fragments thereof.

In relation to a nucleic acid or polypeptide, which are herein components of the bacteriophage, the term *"isolated"* designates e.g., a nucleic acid molecule or polypeptide which is separated from at least some of the components of its natural environment.

*"Sequence identity"* between amino acid or nucleic acid sequences can be determined by comparing a position in each of the sequences which may be aligned for the purposes of comparison. When a position in the compared sequences is occupied by the same nucleotide or amino acid, then the sequences are identical at that position. A degree of identity between amino acid sequences is a function of the number of identical amino acid sequences that are shared between these sequences. A degree of sequence identity between nucleic acids is a function of the number of identical nucleotides at positions shared by these sequences.

To determine the *"percentage of sequence identity"* between two amino acid sequences or two nucleic acid sequences, the sequences are aligned for optimal comparison. For example, gaps can be introduced in the sequence of a first amino acid sequence or a first nucleic acid sequence for optimal alignment with the second amino acid sequence or second nucleic acid sequence. The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percentage (%) of identity between the two sequences is a function of the number of identical positions shared by the sequences. Hence, the percentage of identity can be calculated by multiplying the number of identical positions by 100 and dividing by the length of the aligned region (overlapping positions), including gaps (only internal gaps, not the gaps at the sequence ends). In this comparison, the sequences can be of the same length, or may be of different lengths. Identity scoring only counts perfect matches, and does not consider the degree of similarity of amino acids to one another. The percentage of identity is herein calculated over the entire length of the sequence of reference.

Optimal alignment of sequences may be herein preferably conducted by a global homology alignment algorithm, such as by the algorithm described by Needleman and Wunsch (Journal of Molecular Biology, 1970, 48 (3): 443-53), by computerized implementations of this algorithm, or by visual inspection. A global homology alignment is particularly preferred if the alignment is performed using sequences of the same or similar length. Examples of global homology alignment algorithms suitable for comparing phage genomes are well-known to the skilled practitioner, and include, without limitation, EMBOSS STRETCHER (global alignment).

The term *"subject"* or *"patient"* is used herein to describe any member of the animal kingdom, preferably a mammal, such as human subjects as well as non-human subjects (dogs, cats, horses, ruminants, sheep, goats, pigs, birds, etc.), or any member of the vegetal kingdom. In a preferred embodiment, the subject is a human being, including adult and child, more preferably is a human child.

The term *"sample"* or *"biological sample"* means any sample containing at least cells. Examples of such samples include fluids such as blood, plasma, saliva, or urine as well as biopsies, organs, tissues or cell samples. The sample may be treated prior to its use.

The term *"treatment"* or *"therapy"* designates herein both a curative treatment and/or a prophylactic treatment of a disease. A curative treatment is defined as a treatment resulting in cure or a treatment alleviating, improving and/or eliminating, reducing and/or stabilizing the symptoms of a disease or the suffering that it causes directly or indirectly. A prophylactic treatment comprises both a treatment resulting in the prevention of a disease and a treatment reducing and/or delaying the incidence of a disease or the risk of its occurrence.

The term *"efficacy"* of treatment or *"response"* to a bacteriophage therapy as used herein refers to a treatment which results in a decrease in the number of *E. coli* strain(s) in a subject after bacteriophage treatment when compared to the number *of E. coli* strain(s) before treatment. A *"good responder"* subject refers to a subject who shows or will show a clinically significant recovery when treated with a bacteriophage therapy.

The term *"composition"* or *"cocktail"* of bacteriophages (plural) designates a combination of at least two or more distinct bacteriophages. The bacteriophages in a cocktail/composition are preferably formulated together, i.e., in a same vessel or packaging, although they may be used as kits of parts wherein the (or some of the) bacteriophages are formulated or packaged separately and combined when used or administered.

Additional definitions are provided throughout the specification.

### Description of embodiments

The present invention may be understood more readily by reference to the following detailed description, including preferred embodiments of the invention.

### Bacteriophages

The Inventors have discovered novel virulent bacteriophages which display, either individually or in combination, a remarkable host range spectrum of lytic activity towards *E. coli.* These bacteriophages are unique in that they have been successfully isolated, for the first time, from human intestinal microbiota. Besides, as will be illustrated further, these bacteriophages can be used, alone or in combination, as very potent antibacterial agents, notably as pharmaceutical or veterinary agents, to target a wide spectrum *of E. coli* strains.

More specifically, the following bacteriophages have been selected and characterized by the Inventors. Their corresponding nucleic acid sequences are indicated in the following Table 3.

**Table 3. Bacteriophages according to the invention**

| **SEQ ID number** | **Bacteriophage name** |
|---|---|
| SEQ ID NO: 1 | rV5_ev156 |
| SEQ ID NO: 2 | rV5_ev147 |
| SEQ ID NO: 3 | rV5_ev158 |
| SEQ ID NO: 4 | rV5_ev146 |
| SEQ ID NO: 5 | rV5_ev168 |
| SEQ ID NO: 6 | phAPEC8_ev052 |
| SEQ ID NO: 7 | T5_ev212 |
| SEQ ID NO: 8 | T5_ev219 |
| SEQ ID NO: 9 | VpaE1_ev035 |
| SEQ ID NO: 10 | VpaE1_ev108 |
| SEQ ID NO: 11 | VpaE1_ev078 |
| SEQ ID NO: 12 | T4_ev151 |
| SEQ ID NO: 13 | T4_ev240 |
| SEQ ID NO: 14 | Stevie_ev116 |
| SEQ ID NO: 15 | Gluttony_ev152 |

These bacteriophages have been selected for their potency and combination potential. Their lytic profile has been determined on a broad number of *E. coli* strains. In particular, Table 5, illustrated in the Examples below, highlights their lytic potential on at least 70 human gut *E. coli* strains, which are notably present in children. As immediately apparent from said Table, these phages have individually a very strong lytic power, and can be combined in order to kill all, or at least a broad range, of the tested *E. coli* strains, thereby producing broad spectrum antibacterial compositions (cocktails). As an illustration, a cocktail of all 15 bacteriophages according to the invention could effectively kill all bacteria listed in Table 5. Interestingly, Table 5 reports that all these phages are lytic against at least one *E. coli* strain belonging to the phylogroup B2, which is a phylogroup known to encompass pathogenic *E. coli* strains.

Accordingly, in a first aspect, the present invention relates to a bacteriophage having lytic activity to an *Escherichia coli* strain and having a genome comprising or consisting of a nucleotide sequence selected from any one of SEQ ID NO: 1 to 15 or a nucleotide sequence having at least 97% identity thereto, preferably at least 98% or 99% identity thereto. As explained above, *"at least 97% sequence identity"* should be understood as *"at least 97% sequence identity over the entire length of the sequence of reference",* i.e. herein over the entire length of any one of SEQ ID NO: 1 to 15.

In a preferred embodiment, the bacteriophage according the invention as described herein is lytic against an *E. coli* gut strain and/or against a pathogenic and/or antibiotic-resistant *E. coli* strain. The pathogenic and/or antibiotic-resistant *E. coli* strain may accordingly be an *E. coli* gut strain.

As explained above, the bacteriophage according to the invention is a myophage or a siphophage.

In a preferred embodiment, the bacteriophage according to the invention is a myophage having lytic activity to an *Escherichia coli* strain and having a genome comprising or consisting of a nucleotide sequence selected from any one of SEQ ID NO: 1 to 6 and 9 to 13 or a nucleotide sequence having at least 97% identity thereto, preferably at least 98% or 99% identity thereto.

Still, preferably, the myophage according to the invention is a myophage Neck Type 1 (M 1), such as of cluster 7 (M 1,7) according to the VIRFAM classification, said bacteriophage having lytic activity to an *Escherichia coli* strain and having a genome comprising or consisting of a nucleotide sequence selected from any one of SEQ ID NO: 1 to 6 and 9 to 11 or a nucleotide sequence having at least 97% identity thereto, preferably at least 98% or 99% identity thereto.

Myophages of cluster 7 (M 1,7) typically have between about 60 and 150 genes, and infect mostly the bacterial phylum Proteobacteria. The typical gene organization of the head-to-tail interface of these myophages (M 1,7) has the following order: [TermL-P-x(3-4)-MCP-x(1-3)-Ad1-Ne1-Hc1-Tc1-Sheath] (TermL: Terminase Large subunit; P: Portal; x(3-4): any amino acid at the 3 to 4 next positions; MCP : Major Capsid Protein; Ad1: Adaptator type 1; Ne1: Neck type 1 ; Hc1: Head-closure type 1; Tc1: Tail completion type 1).

For example, in one embodiment, a preferred myophage (M 1,7) according to the invention is a rV5-like bacteriophage, said bacteriophage having lytic activity to an *Escherichia coli* strain and having a genome comprising or consisting of a nucleotide sequence selected from any one of SEQ ID NO: 1 to 5 or a nucleotide sequence having at least 97% identity thereto, preferably at least 98% or 99% identity thereto.

The term *"rV5-like bacteriophage"* or *"rV5-related bacteriophage"* is meant herein to encompass phages belonging to the rV5 species, or in other words, phages having a closely related phenotype to the one of native rV5 bacteriophage (Kropinski al., Virol J, 2013, 10:76), or at least a closely related structure to rV5. The native phage V5 is notably known for its broad-host range on pathogenic O157:H7 strains, a quarter of the ECOR collection, as well as a large panel of pathogenic strains other than serotype O157:H7. In the context of the present invention, the rV5-like bacteriophages were the most prevalent virulent phages identified in infant gut and displayed the broadest host range in *E.coli* (a mean of about 56% of the tested strains were killed) among all virulent phages, thereby suggesting a possible link between infectivity and prevalence for virulent phages in infant gut.

In a particular embodiment, the bacteriophage according to the invention is the rV5-like bacteriophage rV5_ev156, or any variant thereof. The rV5_ev156 bacteriophage, or any variant thereof, can be produced or expanded in e.g., *E. coli* strain C or *E. coli* strain MAC1403 (the latter being a restrictionless derivative of *E. coli* strain MG1655). rV5_ev156, or any variant thereof, has lytic activity against various *E. coli* strains, such as an *E. coli* gut strain, in particular those described in Table 5 below, among which some belong to the *E. coli* phylogroup B2. rV5_ev156, or any variant thereof, has a genome consisting of a sequence as set forth in SEQ ID NO: 1, or a sequence having at least 97% identity, preferably at least 98% and still more preferably 99% identity to SEQ ID NO: 1. It is also provided an isolated nucleic acid sequence from rV5_ev156 bacteriophage, or any variant thereof. The invention also encompasses isolated polypeptides encoded by rV5_ev156 bacteriophage, or any variant thereof, or encoded by an isolated nucleic acid sequence from the rV5_ev156 bacteriophage of the invention. The rV5_ev156 bacteriophage of the invention displays the broadest host-range in *E. coli* among all phages of the invention (phage infectivity on the tested *E. coli* strains: about 73%).

In another particular embodiment, the bacteriophage according to the invention is the rV5-like bacteriophage rV5_ev147, or any variant thereof. The rV5_ev147 bacteriophage, or any variant thereof, can be produced or expanded in e.g., *E. coli* strain C or *E. coli* strain MAC1403. rV5_ev147, or any variant thereof, has lytic activity against various *E. coli* strains, such as an *E. coli* gut strain, in particular those described in Table 5 below, among which some belong to the *E. coli* phylogroup B2. rV5_ev147, or any variant thereof, has a genome consisting of a sequence as set forth in SEQ ID NO: 2, or a sequence having at least 97% identity, preferably at least 98% and still more preferably 99% identity to SEQ ID NO: 2. It is also provided an isolated nucleic acid sequence from rV5_ev147 bacteriophage, or any variant thereof. The invention also encompasses isolated polypeptides encoded by rV5_ev147 bacteriophage, or any variant thereof, or encoded by an isolated nucleic acid sequence from the rV5_ev147 bacteriophage of the invention. The rV5_ev147 bacteriophage of the invention displays one of the broadest host-range in *E. coli* among all phages described herein (phage infectivity on the tested *E. coli* strains: about 72%).

In another particular embodiment, the bacteriophage according to the invention is the rV5-like bacteriophage rV5_ev158, or any variant thereof. The rV5_ev158 bacteriophage, or any variant thereof, can be produced or expanded in e.g., *E. coli* strain C or *E. coli* strain MAC1403. rV5_ev158, or any variant thereof, has lytic activity against various *E. coli* strains, such as an *E. coli* gut strain, in particular those described in Table 5 below, among which some belong to the *E. coli* phylogroup B2. rV5_ev158, or any variant thereof, has a genome consisting of a sequence as set forth in SEQ ID NO: 3, or a sequence having at least 97% identity, preferably at least 98% and still more preferably 99% identity to SEQ ID NO: 3. It is also provided an isolated nucleic acid sequence from rV5_ev158 bacteriophage, or any variant thereof. The invention also encompasses isolated polypeptides encoded by rV5_ev158 bacteriophage, or any variant thereof, or encoded by an isolated nucleic acid sequence from the rV5_ev158 bacteriophage of the invention. The rV5_ev158 bacteriophage of the invention displays one of the broadest host-range in *E. coli* among all phages d of the invention (phage infectivity on the tested *E. coli* strains: about 64%).

In another particular embodiment, the bacteriophage according to the invention is the rV5-like bacteriophage rV5_ev146, or any variant thereof. The rV5_ev146 bacteriophage, or any variant thereof, can be produced or expanded in e.g., *E. coli* strain C or *E. coli* strain MAC1403. rV5_ev146, or any variant thereof, has lytic activity against various *E. coli* strains, such as an *E. coli* gut strain, in particular those described in Table 5 below, among which some belong to the *E. coli* phylogroup B2. rV5_ev146, or any variant thereof, has a genome consisting of a sequence as set forth in SEQ ID NO: 4, or a sequence having at least 97% identity, preferably at least 98% and still more preferably 99% identity to SEQ ID NO: 4. It is also provided an isolated nucleic acid sequence from rV5_ev146 bacteriophage, or any variant thereof. The invention also encompasses isolated polypeptides encoded by rV5_ev146 bacteriophage, or any variant thereof, or encoded by an isolated nucleic acid sequence from the rV5_ev146 bacteriophage of the invention. The rV5_ev146 bacteriophage of the invention displays one of the broadest host-range in *E. coli* among all phages of the invention (phage infectivity on the tested *E. coli* strains: about 45%).

Yet, in another particular embodiment, the bacteriophage according to the invention is the rV5-like bacteriophage rV5_ev168, or any variant thereof. The rV5_ev168 bacteriophage, or any variant thereof, can be produced or expanded in e.g., *E. coli* strain C or *E. coli* strain MAC1403. rV5_ev168, or any variant thereof, has lytic activity against various *E. coli* strains, such as an *E. coli* gut strain, in particular those described in Table 5 below, among which some belong to the *E. coli* phylogroup B2. rV5_ev168, or any variant thereof, has a genome consisting of a sequence as set forth in SEQ ID NO: 5, or a sequence having at least 97% identity, preferably at least 98% and still more preferably 99% identity to SEQ ID NO: 5. It is also provided an isolated nucleic acid sequence from rV5_ev168 bacteriophage, or any variant thereof. The invention also encompasses isolated polypeptides encoded by rV5_ev168 bacteriophage, or any variant thereof, or encoded by an isolated nucleic acid sequence from the rV5_ev168 bacteriophage of the invention. The rV5_ev168 bacteriophage of the invention displays a relatively broad host-range in *E. coli* (phage infectivity on the tested *E. coli* strains: about 26%).

In another embodiment, a preferred myophage (M 1,7) according to the invention is a phAPEC8-like bacteriophage, said bacteriophage having lytic activity to an *Escherichia coli* strain and having a genome comprising or consisting of the nucleotide sequence SEQ ID NO: 6 or a nucleotide sequence having at least 97% identity thereto, preferably at least 98% or 99% identity thereto.

The term *"phAPEC8-like bacteriophage"* or *"phAPEC8-related bacteriophage"* is meant herein to encompass phages belonging to the phAPEC8 species, or in other words, phages having a closely related phenotype to the one of native phAPEC8 bacteriophage (Tsonos et al., J Virol., 2012, 86(23):13117-8), or at least a closely related structure to phAPEC8. The native phAPEC8 phage has been reported to have broad host ranges in the literature. In the context of the present invention, the phAPEC8-like bacteriophage identified manifested a very broad host-range in *E. coli* (about 66% of tested strains killed).

In a particular embodiment, the bacteriophage according to the invention is the phAPEC8-like bacteriophage phAPEC8_ev052, or any variant thereof. The phAPEC8_ev052 bacteriophage, or any variant thereof, can be produced or expanded in e.g., *E. coli* strain C or *E. coli* strain MAC1403. phAPEC8_ev052, or any variant thereof, has lytic activity against various *E. coli* strains, such as an *E. coli* gut strain, in particular those described in Table 5 below, among which some belong to the *E. coli* phylogroup B2. phAPEC8_ev052, or any variant thereof, has a genome consisting of a sequence as set forth in SEQ ID NO: 6, or a sequence having at least 97% identity, preferably at least 98% and still more preferably 99% identity to SEQ ID NO: 6. It is also provided an isolated nucleic acid sequence from phAPEC8_ev052 bacteriophage, or any variant thereof. The invention also encompasses isolated polypeptides encoded by phAPEC8_ev052 bacteriophage, or any variant thereof, or encoded by an isolated nucleic acid sequence from the phAPEC8_ev052 bacteriophage of the invention. The phAPEC8_ev052 bacteriophage of the invention displays one of the broadest host-range in *E. coli* among all phages described herein (phage infectivity on the tested *E. coli* strains: about 66%).

Still, in another embodiment, a preferred myophage (M 1,7) according to the invention is a VpaE1-like bacteriophage, said bacteriophage having lytic activity to an *Escherichia coli* strain and having a genome comprising or consisting of a nucleotide sequence selected from any one of SEQ ID NO: 9 to 11, or a nucleotide sequence having at least 97% identity thereto, preferably at least 98% or 99% identity thereto.

The term *"VpaE1-like bacteriophage"* or "*VpaE1-related bacteriophage"* is meant herein to encompass phages belonging to the VpaE1 species, or in other words, phages having a closely related phenotype to the one of native VpaE1 bacteriophage (Šimoliunas et al., Viruses, 2015, 27, 7(12):6163-81), or at least a closely related structure to VpaE1. The native phage VpaE1 is notably known for its capacity to replicate over a wide range of temperatures (from about 9°C to about 45°C), and for its interesting host range properties, notably its preferential targeting of *E. coli* mutants whose LPS cores are truncated to resist infection by other phages such as T4. The newly isolated VpaE1-like bacteriophages according to the invention display a tail fiber closely related to VpaE1 gp74 protein, which may explain their limited host range in *E. coli* (between about 12% to about 31% of the tested strains were killed).

In a particular embodiment, the bacteriophage according to the invention is the VpaE1-like bacteriophage VpaE1_ev035, or any variant thereof. The VpaE1_ev035 bacteriophage, or any variant thereof, can be produced or expanded in e.g., *E. coli* strain C or *E. coli* strain MAC1403. VpaE1_ev035, or any variant thereof, has lytic activity against various *E. coli* strains, such as an *E. coli* gut strain, in particular those described in Table 5 below, among which some belong to the *E. coli* phylogroup B2. VpaE1_ev035, or any variant thereof, has a genome consisting of a sequence as set forth in SEQ ID NO: 9, or a sequence having at least 97% identity, preferably at least 98% and still more preferably 99% identity to SEQ ID NO: 9. It is also provided an isolated nucleic acid sequence from VpaE1_ev035 bacteriophage, or any variant thereof. The invention also encompasses isolated polypeptides encoded by VpaE1_ev035 bacteriophage, or any variant thereof, or encoded by an isolated nucleic acid sequence from the VpaE1_ev035 bacteriophage of the invention. The VpaE1_ev035 bacteriophage of the invention displays a narrower host-range in *E. coli* than most phages of the invention (phage infectivity on the tested *E. coli* strains: about 12%).

In another particular embodiment, the bacteriophage according to the invention is the VpaE1-like bacteriophage VpaE1_ev108, or any variant thereof. The VpaE1_ev108 bacteriophage, or any variant thereof, can be produced or expanded in e.g., *E. coli* strain C or *E. coli* strain MAC1403. VpaE1_ev108, or any variant thereof, has lytic activity against various *E. coli* strains, such as an *E. coli* gut strain, in particular those described in Table 5 below, among which some belong to the *E. coli* phylogroup B2. VpaE1_ev035, or any variant thereof, has a genome consisting of a sequence as set forth in SEQ ID NO: 10, or a sequence having at least 97% identity, preferably at least 98% and still more preferably 99% identity to SEQ ID NO: 10. It is also provided an isolated nucleic acid sequence from VpaE1_ev108 bacteriophage, or any variant thereof. The invention also encompasses isolated polypeptides encoded by VpaE1_ev108 bacteriophage, or any variant thereof, or encoded by an isolated nucleic acid sequence from the VpaE1_ev108 bacteriophage of the invention. The VpaE1_ev108 bacteriophage of the invention displays a relatively broad host-range in *E. coli* (phage infectivity on the tested *E. coli* strains: about 31%).

In another particular embodiment, the bacteriophage according to the invention is the VpaE1-like bacteriophage VpaE1_ev078, or any variant thereof. The VpaE1_ev078 bacteriophage, or any variant thereof, can be produced or expanded in e.g., *E. coli* strain C or *E. coli* strain MAC1403. VpaE1_ev078, or any variant thereof, has lytic activity against various *E. coli* strains, such as an *E. coli* gut strain, in particular those described in Table 5 below, among which some belong to the *E. coli* phylogroup B2. VpaE1_ev078, or any variant thereof, has a genome consisting of a sequence as set forth in SEQ ID NO: 11, or a sequence having at least 97% identity, preferably at least 98% and still more preferably 99% identity to SEQ ID NO: 11. It is also provided an isolated nucleic acid sequence from VpaE1_ev078 bacteriophage, or any variant thereof. The invention also encompasses isolated polypeptides encoded by VpaE1_ev078 bacteriophage, or any variant thereof, or encoded by an isolated nucleic acid sequence from the VpaE1_ev078 bacteriophage of the invention. The VpaE1_ev078 bacteriophage of the invention displays a narrower host-range in *E. coli* than most phages of the invention (phage infectivity on the tested *E. coli* strains: about 16%).

In another preferred embodiment, the myophage according to the invention is a myophage Neck Type 2 (M 2) according to the VIRFAM classification, said bacteriophage having lytic activity to an *Escherichia coli* strain and having a genome comprising or consisting of a nucleotide sequence selected from any one of SEQ ID NO: 12 to 13 or a nucleotide sequence having at least 97% identity thereto, preferably at least 98% or 99% identity thereto.

For example, in one embodiment, a preferred myophage (M 2) according to the invention is a T4-like bacteriophage, said bacteriophage having lytic activity to an *Escherichia coli* strain and having a genome comprising or consisting of a nucleotide sequence selected from any one of SEQ ID NO: 12 to 13 or a nucleotide sequence having at least 97% identity thereto, preferably at least 98% or 99% identity thereto.

The term "*T4-like bacteriophage"* or "*T4-related bacteriophage"* is meant herein to encompass phages belonging to the T4 species, or in other words, phages having a closely related phenotype to the one of native T4 bacteriophage (Yap et al., Future Microbiol., 2014, 9: 1319-1327), or at least a closely related structure to T4. Known phages of the T4 species are prevalent in the mammalian gut, and have been used successfully in human phage therapy trials against acute bacterial infection (Sarker et al., Ebiomedicine, 2016, 4: 124-137). The newly isolated T4-like bacteriophages according to the invention are most closely related to two well-known variants of T4, namely RB32 and RB38 (Petrov et al., Virology Journal, 2010, 7:292), and, interestingly, do not display the same host range in *E. coli* (about 14% and about 45% of the tested strains were killed, respectively).

In a particular embodiment, the bacteriophage according to the invention is the T4-like bacteriophage T4_ev151, or any variant thereof. The T4_ev151 bacteriophage, or any variant thereof, can be produced or expanded in e.g., *E. coli* strain C or *E. coli* strain MAC1403. T4_ev151, or any variant thereof, has lytic activity against various *E. coli* strains, such as an *E. coli* gut strain, in particular those described in Table 5 below, among which some belong to the *E. coli* phylogroup B2. T4_ev151, or any variant thereof, has a genome consisting of a sequence as set forth in SEQ ID NO: 12, or a sequence having at least 97% identity, preferably at least 98% and still more preferably 99% identity to SEQ ID NO: 12. It is also provided an isolated nucleic acid sequence from T4_ev151 bacteriophage, or any variant thereof. The invention also encompasses isolated polypeptides encoded by T4_ev151 bacteriophage, or any variant thereof, or encoded by an isolated nucleic acid sequence from the T4_ev151 bacteriophage of the invention. The T4_ev151 bacteriophage of the invention displays a broad host-range in *E. coli* among all phages of the invention (phage infectivity on the tested *E. coli* strains: about 45%).

In another particular embodiment, the bacteriophage according to the invention is the T4-like bacteriophage T4_ev240, or any variant thereof. The T4_ev240 bacteriophage, or any variant thereof, can be produced or expanded in e.g., *E. coli* strain C or *E. coli* strain MAC1403. T4_ev240, or any variant thereof, has lytic activity against various *E. coli* strains, such as an *E. coli* gut strain, in particular those described in Table 5 below, among which some belong to the *E. coli* phylogroup B2. T4_ev240, or any variant thereof, has a genome consisting of a sequence as set forth in SEQ ID NO: 13, or a sequence having at least 97% identity, preferably at least 98% and still more preferably 99% identity to SEQ ID NO: 13. It is also provided an isolated nucleic acid sequence from T4_ev240 bacteriophage, or any variant thereof. The invention also encompasses isolated polypeptides encoded by T4_ev240 bacteriophage, or any variant thereof, or encoded by an isolated nucleic acid sequence from the T4_ev240 bacteriophage of the invention. The T4_ev240 bacteriophage of the invention displays a narrower host-range in *E. coli* than most phages of the invention (phage infectivity on the tested *E. coli* strains: about 14%).

In another preferred embodiment, the bacteriophage according to the invention is a siphophage having lytic activity to an *Escherichia coli* strain and having a genome comprising or consisting of a nucleotide sequence selected from any one of SEQ ID NO: 7 to 8 and 14 to 15 or a nucleotide sequence having at least 97% identity thereto, preferably at least 98% or 99% identity thereto.

In a particularly preferred embodiment, the siphophage according to the invention is a siphophage Neck Type 1 (S 1), such as of cluster 4 (S 1,4) or 5 (S 1,5) according to the VIRFAM classification, said bacteriophage having lytic activity to an *Escherichia coli* strain and having a genome comprising or consisting of a nucleotide sequence selected from any one of SEQ ID NO: 7 to 8 and 14 to 15 or a nucleotide sequence having at least 97% identity thereto, preferably at least 98% or 99% identity thereto.

Siphophages (S 1,4) generally infect the same class of bacteria, namely Actinobacteria, except phage T5 which infects Gammaproteobacteria. For about half of these phages, genes encoding both Hc1 and the MCP or only Hc1 (phage T5) were not detected via the VIRFAM platform. Typically, one half of these phages exhibit a lot of inserted genes between the Portal and Ad1 genes, and Ne1 is systematically positioned after the gene Tc1 in their genome, while the other half of phages display the tight [TermL-x(0-1)-P-x(1-2)-MCP-x(0-1)-Ad1-Hc1-Ne1-Tc1] gene head-to-tail scheme.

Siphophages (S 1,5) mostly infect Gammaproteobacteria. Their genome organization follows the gene organization TermL-Portal-x(2-4)-MCP-x(0-1)-Ad1-Hc1-Ne1-Tc1. The position of the gene Ne1 is versatile with half of phages displaying an unusual/atypical gene position after the gene MTP (Major Tail Protein).

For example, in one embodiment, a preferred siphophage (S 1,4) according to the invention is a T5-like bacteriophage, said bacteriophage having lytic activity to an *Escherichia coli* strain and having a genome comprising or consisting of the nucleotide sequence SEQ ID NO: 7 or 8, or a nucleotide sequence having at least 97% identity thereto, preferably at least 98% or 99% identity thereto.

The term *"T5-like bacteriophage"* or "*T5-related bacteriophage"* is meant herein to encompass phages belonging to the T5 species, or in other words, phages having a closely related phenotype to the one of native T5 bacteriophage (Zivanovic et al., J Virol., 2014, 88(2):1162-1174; Sayers J. Bacteriophage T5. In: Calendar R (ed) The bacteriophages, 2nd edn. 2006, Oxford University Press, Oxford, pp 268-276), or at least a closely related structure to T5. Phages of the T5 species are frequently found in the fecal samples of mammals. Among its particularities, the native T5 bacteriophage encodes either one of two receptor-binding proteins allowing two different bacterial receptors to be recognized (FhuA and BtuB), as well as various types of L shaped tail fibers which help to overcome the bacterial surface shielding by O-glycosylated sugars. The genome of the newly isolated T5-like bacteriophages according to the invention show good overall synteny to native T5, except at two loci: the locus of the gene encoding the phage receptor protein for the BtuB bacterial receptor, and the locus of the gene encoding the L-shape tail fiber proteins. These slight differences may explain the difference in *E. coli* host-range observed between these new T5-like bacteriophages. Indeed, a marked gradation of strain infectivity was observed for native phage T5 (about 8% of the *E. coli* strains infected) compared to T5_ev219 and T5_ev212 (about 29% and about 43% of the strains infected, respectively).

In a particular embodiment, the bacteriophage according to the invention is the T5-like bacteriophage T5_ev212, or any variant thereof. The T5_ev212 bacteriophage, or any variant thereof, can be produced or expanded in e.g., *E. coli* strain C or *E. coli* strain MAC1403. T5_ev212, or any variant thereof, has lytic activity against various *E. coli* strains, such as an *E. coli* gut strain, in particular those described in Table 5 below, among which some belong to the *E. coli* phylogroup B2. T5_ev212, or any variant thereof, has a genome consisting of a sequence as set forth in SEQ ID NO: 7, or a sequence having at least 97% identity, preferably at least 98% and still more preferably 99% identity to SEQ ID NO: 7. It is also provided an isolated nucleic acid sequence from T5_ev212 bacteriophage, or any variant thereof. The invention also encompasses isolated polypeptides encoded by T5_ev212 bacteriophage, or any variant thereof, or encoded by an isolated nucleic acid sequence from the T5_ev212 bacteriophage of the invention. The T5_ev212 bacteriophage of the invention displays a broad host-range in *E. coli* among all phages of the invention (phage infectivity on the tested *E. coli* strains: about 43%).

In another particular embodiment, the bacteriophage according to the invention is the T5-like bacteriophage T5_ev219, or any variant thereof. The T5_ev219 bacteriophage, or any variant thereof, can be produced or expanded in e.g., *E. coli* strain C or *E. coli* strain MAC1403. T5_ev219, or any variant thereof, has lytic activity against various *E. coli* strains, such as an *E. coli* gut strain, in particular those described in Table 5 below, among which some belong to the *E. coli* phylogroup B2. T5_ev219, or any variant thereof, has a genome consisting of a sequence as set forth in SEQ ID NO: 8, or a sequence having at least 97% identity, preferably at least 98% and still more preferably 99% identity to SEQ ID NO: 8. It is also provided an isolated nucleic acid sequence from T5_ev219 bacteriophage, or any variant thereof. The invention also encompasses isolated polypeptides encoded by T5_ev219 bacteriophage, or any variant thereof, or encoded by an isolated nucleic acid sequence from the T5_ev219 bacteriophage of the invention. The T5_ev219 bacteriophage of the invention displays a relatively broad host-range in *E. coli* among all phages of the invention (phage infectivity on the tested *E. coli* strains: about 29%).

Yet, in another embodiment, a preferred siphophage (S 1) according to the invention is a Stevie-like bacteriophage, said bacteriophage having lytic activity to an *Escherichia coli* strain and having a genome comprising or consisting of the nucleotide sequence SEQ ID NO: 14 or a nucleotide sequence having at least 97% identity thereto, preferably at least 98% or 99% identity thereto.

The term *"Stevie-like bacteriophage"* or *"Stevie-related bacteriophage"* is meant herein to encompass phages belonging to the Stevie species, or in other words, phages having a closely related phenotype to the one of native Stevie bacteriophage (Shaw et al., Genome Announc., 2015, 3(1): e01434-14), or at least a closely related structure to Stevie. Stevie is related to the virulent siphophage T1 in that it has an abundance of rho-independent terminators, a distinguishing feature of T1-like phages, with 21 predicted in its genome compared to the 17 found in phage T1.

Thus, in a particular embodiment, the bacteriophage according to the invention is the Stevie-like bacteriophage Stevie_ev116, or any variant thereof. The Stevie_ev116 bacteriophage, or any variant thereof, can be produced or expanded in e.g., *E. coli* strain C or *E. coli* strain MAC1403. Stevie_ev116, or any variant thereof, has lytic activity against various *E. coli* strains, such as an *E. coli* gut strain, in particular those described in Table 5 below, among which some belong to the *E. coli* phylogroup B2. Stevie_ev116, or any variant thereof, has a genome consisting of a sequence as set forth in SEQ ID NO: 14, or a sequence having at least 97% identity, preferably at least 98% and still more preferably 99% identity to SEQ ID NO: 14. It is also provided an isolated nucleic acid sequence from Stevie ev212 bacteriophage, or any variant thereof. The invention also encompasses isolated polypeptides encoded by Stevie_ev116 bacteriophage, or any variant thereof, or encoded by an isolated nucleic acid sequence from the Stevie_ev116 bacteriophage of the invention. The Stevie_ev116 bacteriophage of the invention displays a narrower host-range in *E. coli* than most phages of the invention (phage infectivity on the tested *E. coli* strains: about 13%).

Still, in another embodiment, a preferred siphophage (S 1,5) according to the invention is a Gluttony-like bacteriophage, said bacteriophage having lytic activity to an *Escherichia coli* strain and having a genome comprising or consisting of the nucleotide sequence SEQ ID NO: 15 or a nucleotide sequence having at least 97% identity thereto, preferably at least 98% or 99% identity thereto.

The term *"Gluttony-like bacteriophage"* or *"Gluttony-related bacteriophage"* is meant herein to encompass phages belonging to the Gluttony species, or in other words, phages having a closely related phenotype to the one of native Gluttony bacteriophage (Malki al., Genome Announc., 2016, 4(6): e01003-16), or at least a closely related structure to Gluttony. Gluttony is an *E. coli* bacteriophage isolated from the microbiota of a female bladder.

Thus, in a particular embodiment, the bacteriophage according to the invention is the Gluttony-like bacteriophage Gluttony_ev152, or any variant thereof. The Gluttony_ev152 bacteriophage, or any variant thereof, can be produced or expanded in e.g., *E. coli* strain C or *E. coli* strain MAC1403. Gluttony_ev152, or any variant thereof, has lytic activity against various *E. coli* strains, such as an *E. coli* gut strain, in particular those described in Table 5 below, among which some belong to the *E. coli* phylogroup B2. Gluttony_ev152, or any variant thereof, has a genome consisting of a sequence as set forth in SEQ ID NO: 15, or a sequence having at least 97% identity, preferably at least 98% and still more preferably 99% identity to SEQ ID NO: 15. It is also provided an isolated nucleic acid sequence from Gluttony_ev152 bacteriophage, or any variant thereof. The invention also encompasses isolated polypeptides encoded by Gluttony_ev152 bacteriophage, or any variant thereof, or encoded by an isolated nucleic acid sequence from the Gluttony_ev152 bacteriophage of the invention. The Gluttony_ev152 bacteriophage of the invention displays a narrower host-range in *E. coli* than most phages of the invention (phage infectivity on the tested *E. coli* strains: about 13%).

The bacteriophages according the invention can be prepared by standard culture, isolation and purification methods which are well-known in the art. For example, *E. coli* bacteria can be cultured and infected by a sample of a bacteriophage, and then treated to remove bacterial cells and debris. To do so, an enriched bacteriophage solution can be plated in a suitable medium, for example agar medium, with embedded susceptible host strains of *E. coli* to obtain plaques. In the context of the present invention, particularly preferred *E. coli* suitable to produce the bacteriophages described herein are the *E. coli* strain C or *E. coli* strain MAC1403. Then, single plaque can be picked out for subsequent bacteriophage purification and amplification. One or more cycles of selective amplification of bacteriophages of the invention may be performed, for example by mixing bacteriophages with the competent *E*. *coli,* followed by addition of a growth medium and incubation at selected test growing conditions. Following centrifugation, the cleared amplified supernatant can be filtered through filter and subjected to another cycle of selective amplification or tested for presence of lytic activity. The titer of phage in a suspension and the visualization of plaque morphology of bacteriophages of the invention can be estimated by known methods, for example by plaque counting. Additionally, processing bacteriophages of the invention in various purification state (partially purified such as a phage lysate, or completely purified i.e. devoid of lysed bacterial cells), and/or forms (liquid, lyophilized, etc.) for short-, long-, freeze- or any other kind of storage can be carried out by any suitable method as it is well-known in the art (see Clark, 1962).

### Nucleic acids and polypeptides

In another aspect, the invention relates to a nucleic acid isolated from a bacteriophage of the invention, or any functional fragment of such a nucleic acid.

In the present context, the isolated nucleic acid of the invention is preferably capable of lytic activity against *E. coli.* Examples of such nucleic acid include, *inter alia,* nucleic acids encoding an endolysin.

A particularly preferred nucleic acid according to the invention is lytic against an *E. coli* gut strain, and/or against a pathogenic and/or antibiotic-resistant *E. coli* strain. The pathogenic and/or antibiotic-resistant *E. coli* strain may accordingly be an *E. coli* gut strain.

The term "*fragment*" of a nucleic acid typically designates a fragment having at least 10 consecutive nucleotides of said nucleic acid, more preferably at least 15, 20, 25, 30, 35, 40, 50, 100, 1000, 5000 or more consecutive nucleotides of said nucleic acid.

The term "*functional fragment"* of a nucleic acid designates a fragment thereof containing, or consisting of, at least one open reading frame and which is capable of retaining all or part of the activity of the polypeptide encoded by the nucleic acid from which it derives. A preferred functional fragment of a nucleic acid according to the invention is capable of lysing *E. coli.*

The nucleic acid according to the invention can be isolated from the deposited bacteriophages described herein, or produced using recombinant DNA technology (e.g., polymerase chain reaction (PCR) amplification, cloning), enzymatic or chemical synthesis, or combinations thereof, according to general techniques well-known in the art. Also included are homologous sequences and fragments thereof including, but not limited to, natural allelic variants and modified nucleic acid sequences in which nucleotides have been inserted, deleted, substituted, and/or inverted.

In a particular embodiment, the invention relates to a nucleic acid comprising or consisting of a sequence selected from any one of SEQ ID NO: 1 to 15, or a sequence having at least 97%, preferably at least 98%, more preferably 99% or more sequence identity thereto.

In another particular embodiment, the invention relates to a nucleic acid comprising or consisting of a sequence fragment from any one of SEQ ID NO: 1 to 15, or a sequence fragment having at least at least 97%, preferably at least 98%, more preferably 99% or more sequence identity thereto, said fragment comprising an open reading frame and/or a regulatory element such as a promoter.

The nucleic acids of the invention can be in a free form or cloned into a vector, and/or may be used as antibacterial agents *in vivo* or *in vitro.*

In a further aspect, the invention also relates to a polypeptide isolated from a bacteriophage of the invention, or any functional fragment of such a polypeptide.

In the present context, the isolated polypeptide of the invention is preferably lytic towards *E. coli.* Examples of such polypeptide include, *inter alia,* endolysins.

A particularly preferred polypeptide according to the invention is lytic against an *E. coli* gut strain, and/or against a pathogenic and/or antibiotic-resistant *E. coli* strain. The pathogenic and/or antibiotic-resistant *E. coli* strain may accordingly be an *E. coli* gut strain.

The term "*fragment*" of a polypeptide typically designates a fragment having at least 5 consecutive amino acids of said polypeptide, more preferably at least 10, 15, 20, 30, 40, 50 or 100 or more consecutive nucleotides of said polypeptide.

The term "*functional fragment"* with regard to a polypeptide designates a fragment containing, or consisting of, at least a portion of said polypeptide and which retains all or part of the activity of said polypeptide. A preferred functional fragment of a polypeptide according to invention is lytic against *E. coli.*

In a particular embodiment, the invention thus relates to an isolated polypeptide encoded by a nucleic acid comprising or consisting of a sequence selected from any one of SEQ ID NO: 1 to 15, or a sequence having at least 97%, preferably at least 98%, more preferably 99% or more sequence identity thereto.

In another particular embodiment, the invention relates to an isolated polypeptide encoded by a nucleic acid comprising or consisting of a sequence fragment from any one of SEQ ID NO: 1 to 15, or a sequence fragment having at least at least 97%, preferably at least 98%, more preferably 99% or more sequence identity thereto, said fragment comprising an open reading frame and/or a regulatory element such as a promoter.

Polypeptides may be produced by techniques well-known in the art such as synthesis, recombinant technology, or combinations thereof. These polypeptides may also be isolated or purified, and used as antibacterial agents *in vivo* or *in vitro.*

### Antibacterial compositions

A further aspect of the present invention relates to compositions lytic against *E. coli* and comprising at least one of the bacteriophages as described above, preferably at least two of said bacteriophages or more and, optionally, a pharmaceutically or veterinary acceptable excipient. Indeed, the bacteriophages of the invention display a remarkable host range spectrum of lytic activity towards *E. coli* strains. As such, combinations of these bacteriophages may be produced to expand the host spectrum and produce highly effective antibacterial compositions, to notably target a wide spectrum *of E. coli* strains.

For example, a particularly preferred antibacterial composition according to the invention is lytic against an *E. coli* gut strain, and/or against a pathogenic and/or antibiotic-resistant *E. coli* strain. The pathogenic and/or antibiotic-resistant *E. coli* strain may accordingly be an *E. coli* gut strain.

In a preferred embodiment, the invention relates to an antibacterial composition comprising at least one bacteriophage having lytic activity against an *E. coli* strain, said bacteriophage being selected from the bacteriophage having a genome comprising or consisting of a nucleotide sequence of any one of SEQ ID NO: 1 to 15 or a sequence having at least 97% identity thereto, preferably at least 98% or 99% identity thereto.

For example, the antibacterial composition according to the invention can comprise at least one bacteriophage selected from rV5_ev156, rV5_ev147, rV5_ev158, rV5_ev146, rV5_ev168, phAPEC8_ev052, T5_ev212, T5_ev219, VpaE1_ev035, VpaE1_ev108, VpaE1_ev078, T4_ev151, T4_ev240, Stevie_ev116, and/or Gluttony_ev152, and variants thereof, as described above.

Compositions of the invention may comprise at least 2, 3, 4, 5, 6, 7, 8, 9, 10, 11, 11, 12, 13, 14 or all of the 15 distinct bacteriophages as described herein.

Thus, in a preferred embodiment, the invention relates to an antibacterial composition comprising at least two distinct bacteriophages having lytic activity against an *E. coli* strain, said at least two bacteriophages being selected from the bacteriophages having a genome comprising or consisting of a nucleotide sequence of any one of SEQ ID NO: 1 to 15, such as SEQ ID NO: 1 and 12, or a sequence having at least 90% identity thereto, preferably at least 91%, 92%, 93%, 94%, 95%, 96%, 97%, 98% or 99% identity thereto. By *"distinct"* bacteriophages, it is meant herein that said bacteriophages have preferably a distinct host-range with regard to *E. coli* strains, albeit with a possible partial overlap.

For example, the antibacterial composition according to the invention can comprise at least two distinct bacteriophages selected from rV5_ev156, rV5_ev147, rV5_ev158, rV5_ev146, rV5_ev168, phAPEC8_ev052, T5_ev212, T5_ev219, VpaE1_ev035, VpaE1_ev108, VpaE1_ev078, T4_ev151, T4_ev240, Stevie_ev116, and/or Gluttony_ev152, and variants thereof, as described above, such as rV5_ev156 and T4_ev151. A composition comprising at least the two bacteriophages rV5_ev156 and T4_ev151 is particularly useful as it could kill about 80% of the *E. coli* strains of Table 5.

Yet, in a preferred embodiment, the antibacterial composition of the invention comprises at least three, even more preferably at least four distinct bacteriophages, still more preferably at least five distinct bacteriophages, still more preferably at least six distinct bacteriophages, more preferably at least seven distinct bacteriophages, more preferably at least eight distinct bacteriophages, still more preferably at least nine distinct bacteriophages and still more preferably at least ten distinct bacteriophages having lytic activity against an *E*. *coli* strain, said bacteriophage being selected from the bacteriophages having a genome comprising or consisting of a nucleotide sequence of any one of SEQ ID NO: 1 to 15, such as any one of SEQ ID NO: 1, 3, 6, 10 and/or 12, or a sequence having at least 90% identity thereto, preferably at least 91%, 92%, 93%, 94%, 95%, 96%, 97%, 98% or 99% identity thereto.

For example, the antibacterial composition according to the invention can comprise at least three distinct bacteriophages, more preferably at least four distinct bacteriophages, still more preferably at least five distinct bacteriophages, still more preferably at least six distinct bacteriophages, more preferably at least seven distinct bacteriophages, more preferably at least eight distinct bacteriophages, still more preferably at least nine distinct bacteriophages and still more preferably at least ten distinct bacteriophages selected from rV5_ev156, rV5_ev147, rV5_ev158, rV5_ev146, rV5_ev168, phAPEC8_ev052, T5_ev212, T5_ev219, VpaE1_ev035, VpaE1_ev108, VpaE1_ev078, T4_ev151, T4_ev240, Stevie_ev116, and/or Gluttony_ev152, and variants thereof, as described above, such as rV5_ev156, T4_ev151, rV5_ev168, VpaE1_ev108, rV5_ev158 and phAPEC8_ev052. A composition comprising at least the six bacteriophages rV5_ev156, T4_ev151, rV5_ev168, VpaE1_ev108, rV5_ev158 and phAPEC8_ev052 is particularly useful as it could kill about 95 % of the *E. coli* strains of Table 5.

Yet, in a preferred embodiment, the antibacterial composition of the invention comprises a combination of 15 distinct bacteriophages having lytic activity against an *E. coli* strain and having a genome comprising or consisting of a nucleotide sequence of any one of SEQ ID NO: 1 to 15 or a sequence having at least 90% identity thereto, preferably at least 91%, 92%, 93%, 94%, 95%, 96%, 97%, 98% or 99% identity thereto.

More specifically, said antibacterial composition can comprise:
- a bacteriophage having lytic activity against an *E. coli* strain, said bacteriophage having a genome comprising or consisting of a nucleotide sequence of SEQ ID NO: 1 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 2 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 3 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 4 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 5 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 6 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 7 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 8 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 9 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 10 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 11 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 12 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 13 or a sequence having at least 90% identity thereto, preferably at least 91%, 92%, 93%, 94%, 95%, 96%, 97%, 98% or 99% identity thereto;
- a bacteriophage having lytic activity against an *E. coli* strain, said bacteriophage having a genome comprising or consisting of a nucleotide sequence of SEQ ID NO: 14 or a sequence having at least 90% identity thereto, preferably at least 91%, 92%, 93%, 94%, 95%, 96%, 97%, 98% or 99% identity thereto; and
- a bacteriophage having lytic activity against an *E. coli* strain, said bacteriophage having a genome comprising or consisting of a nucleotide sequence of SEQ ID NO: 15 or a sequence having at least 90% identity thereto, preferably at least 91%, 92%, 93%, 94%, 95%, 96%, 97%, 98% or 99% identity thereto.

For example, the antibacterial composition according to the invention can comprise a combination of all of the bacteriophages rV5_ev156, rV5_ev147, rV5_ev158, rV5_ev146, rV5_ev168, phAPEC8_ev052, T5_ev212, T5_ev219, VpaE1_ev035, VpaE1_ev108, VpaE1_ev078, T4_ev151, T4_ev240, Stevie_ev116, and Gluttony_ev152, or variants thereof. Such a composition could indeed kill 100% of the 70 *E. coli* gut strains of Table 5 below (see also Figure 3C).

The composition of the invention exerts lytic activity towards bacterial *E. coli* which may be pathogenic. As such, the composition of the invention may further comprise additional antibacterial agents, such as other bacteriophages known in the art that have complementary and/or similar host specificity to the bacteriophage of the present invention.

It is desired in an aspect of the invention that the composition comprises between 10^{e2} and 10^{e12} PFU of at least one bacteriophage of the invention, preferably between 10^{e5} and 10^{e10} PFU. When the antibacterial composition comprises several bacteriophages as defined above, it is preferred that the composition comprises between 10^{e2} and 10^{e12} PFU of each present bacteriophage of the invention.

The composition of the invention may comprise any effective amount of the selected bacteriophage(s). Preferably, said composition comprises between 10^{e2} and 10^{e12} PFU of each of said bacteriophages, preferably between 10^{e5} and 10^{e10} PFU. The relative amounts of each type of bacteriophage in a composition of the invention may be easily adjusted by a skilled artisan. Typically, when the antibacterial composition comprises several (n) distinct bacteriophages as defined above, the total relative amount %A of each bacteriophage in the composition is more preferably %A= (100/ni)xV, wherein ni represents the number of distinct types of bacteriophages and V is a variability factor comprised between 0.2 and 5. Most preferably, V is comprised between 0.3 and 3, even more preferably between 0.5 and 2, generally between 0.8 and 1.5. In a typical embodiment, when the antibacterial composition comprises several bacteriophages as defined above, it is preferred that the composition comprises between 10^{e2} and 10^{e12} PFU of each present bacteriophage of the invention. Preferably, each type of bacteriophage is present in a composition of the invention in approximately equal relative amounts.

The antibacterial composition of the invention may be in various forms, such as liquid, semi-liquid, solid or lyophilized formulations. To do so, the composition may preferably comprise a suitable diluent or carrier, such as a pharmaceutically or veterinary acceptable excipient or carrier. Examples of suitable excipient or carrier according to the invention include, without limitation, thickeners, diluents, buffers, preservatives, surface active agents and the like. These includes physiologically acceptable solutions or vehicles that are harmless or do not cause any significant specific or non-specific immune reaction to an organism or do not abrogate the biological activity of the bacteriophage. For liquid formulation, saline, sterile water, Ringer's solution, buffered physiological saline, albumin infusion solution, dextrose solution, maltodextrin solution, glycerol, ethanol, and mixtures thereof may be used as a pharmaceutically or veterinary acceptable excipient or carrier. If appropriate, other conventional additives such as antioxidants and bacteriostatic agents may be added. Further excipients or carriers may be additionally added to the composition to prepare injectable formulations such as aqueous solutions, suspensions, and emulsions, oral formulations such as pills, capsules, granules, or tablets, or powdered formulations. Formulations for topical administration may include, band aids, dressings, patches, films, ointments, lotions, creams, gels, drops, suppositories, sprays, nebulizer, tampons, sanitary towels, liquids and powders. Formulations for decontamination or for medical use may also include aerosols or sprays.

Another aspect of the present invention relates to an antibacterial composition comprising at least one, preferably at least two or more distinct isolated nucleic acids or distinct isolated polypeptides of the bacteriophages as described herein, and, optionally, a pharmaceutically or veterinary acceptable excipient.

A further aspect of the invention is directed to a method for preparing a composition, comprising separately producing said one or more bacteriophages, nucleic acids or polypeptides, and combining said bacteriophages, nucleic acids or polypeptides, with a suitable carrier or excipient.

### Antibacterial applications

The bacteriophages of the invention may be used in the medical field, including the human or veterinary medical areas, for example in the treatment of an infection in a subject in need thereof or for improving a subject's condition. To do so, said bacteriophages may either be used individually, or in combination with other bacteriophages. Any functional part of said bacteriophages (i.e. lytic), such as an isolated nucleic acid or isolated polypeptide thereof, may also be used for such purpose.

Accordingly, a further aspect of the present invention relates to a bacteriophage, a nucleic acid, a polypeptide, or an antibacterial composition as described herein, for use as a medicament. In particular, the present invention relates to the use of said bacteriophage, nucleic acid, polypeptide, or antibacterial composition, for the preparation of a medicament. More precisely, the invention provides a method for treating a subject in need thereof, comprising the administration of a therapeutically effective amount of said bacteriophage, nucleic acid, polypeptide, or antibacterial composition to said subject.

The bacteriophages of the invention display more particularly a remarkable host range spectrum of lytic activity towards *E. coli,* notably towards *E. coli* strains present in intestinal microbiota (i.e. enterobacteria). As such, these bacteriophages, or components thereof, may be used to treat diarrhea, gastroenteritis, or other *E. coli* infections such as UTI (urinary tract infections), respiratory tract infections, infections related to burns or wounds, as well as post-surgical infections.

Thus, in a preferred embodiment, the present invention relates to a bacteriophage, a nucleic acid, a polypeptide, or an antibacterial composition as described herein, for use in the treatment of an *E. coli* infection. In particular, the present invention relates to the use of said bacteriophage, nucleic acid, polypeptide, or antibacterial composition, for the preparation of a medicament for treating an *E. coli* infection. More precisely, the invention provides a method for treating an *E. coli* infection in a subject in need thereof, comprising the administration of a therapeutically effective amount of said bacteriophage, nucleic acid, polypeptide, or antibacterial composition to said subject.

The bacteriophages of the invention may also be used for improving the condition of a subject by modifying the microbial flora in said subject. In particular, these bacteriophages, or components thereof, could specifically remove *E. coli* strains in mucous membranes of a subject, such as those present in the gut, thereby modifying its microbial flora and restoring a proper balance. Imbalanced flora dominated by *E. coli* can notably be observed in subjects suffering from diarrhea.

Accordingly, in a preferred embodiment, the present invention relates to a bacteriophage, a nucleic acid, a polypeptide, or an antibacterial composition as described herein, for use for restoring the microbial flora in a subject. In particular, the present invention relates to the use of said bacteriophage, nucleic acid, polypeptide, or antibacterial composition, for the preparation of a medicament for restoring the microbial flora in a subject. More precisely, the invention provides a method for restoring the microbial flora in a subject in need thereof, comprising the administration of a therapeutically effective amount of said bacteriophage, nucleic acid, polypeptide, or antibacterial composition to said subject.

In the context of the above medical applications, the bacteriophages of the invention, or components thereof, may be administered by any convenient route, including intravenous, oral, transdermal, subcutaneous, mucosal, intramuscular, intrapulmonary, intranasal, parenteral, rectal, vaginal and topical. Such administration may be performed directly or indirectly, e.g., via a support. In this regard, the bacteriophages, or components thereof, for example, be applied or sprayed to the afflicted area, for example the skin or a wound. The dosage suitable for applying, spraying, or administrating the bacteriophages of the invention, or components thereof, can be adjusted by the skilled person depending on a variety of factors including formulation, mode of administration, age, weight, sex, condition, diet of the subject being treated at the time of administration, route of administration and reaction sensitivity. A physician having ordinary skills in the art can readily determine and prescribe the therapeutically effective amount required.

The dosing can also be adjusted by the skilled person so that a lytic activity against *E*. *coli* strains is obtained. An efficient dose to obtain a lytic activity *in vivo* typically includes a concentration of at least 10^{e2} PFU/ml, preferably from about 10^{e2} to 10^{e12} PFU/ml, depending on the administration route.

Administration may be performed only once or, if needed, repeated.

The use of the bacteriophages of the present invention is not limited to *in vivo* applications, as it is capable of killing *E. coli* in any environment, including *in vitro.*

Accordingly, another aspect of the invention is to provide an *in vitro* use of a bacteriophage, a nucleic acid, a polypeptide, or an antibacterial composition as described herein, for decontaminating a product contaminated with *E. coli.* Indeed, due to their potent antibacterial effect, the bacteriophages of the invention, or components thereof, can be used as decontaminating agent, to eliminate or at least cause a reduction in bacterial numbers on a product. Such methods may be applied for the treatment of a variety of biological or non-biological products in both medical and non-medical contexts, including solid materials or devices such as, for example, those intended for surgery (e.g. implants), contact lenses, pipes, ducts, laboratory vessels, textiles, food products, etc., or even isolated biological samples. Examples of isolated biological samples include, without limitation, isolated fecal samples. Such fecal samples may indeed need to be decontaminated prior to their transplantation in a subject in need thereof.

The skill person in the art will readily understand that combining several bacteriophages of the invention can destroy a wider range of *E. coli* strains, even *in vivo,* than individual bacteriophages. Accordingly, at least two distinct bacteriophages, nucleic acids or polypeptides of the invention, or more, can preferably be used as a medicament, notably for treating an *E. coli* infection, for restoring the microbial flora in a subject, or for decontaminating a product contaminated with *E. coli.* Whether used in the context of an *in vivo* or an *in vitro* application, said bacteriophages, nucleic acids or polypeptides may be administered simultaneously, separately or sequentially from one another.

Thus, it is a further aspect of the present invention to provide at least two distinct bacteriophages, at least two distinct nucleic acids, or at least two distinct polypeptides as described herein, as a combined preparation for simultaneous, separate or sequential use in therapy, such as in a treatment of *E. coli* infections or for restoring the microbial flora in a subject. It should be understood that preferred combined preparations of at least two (or more) distinct bacteriophages are as described for antibacterial compositions.

It is a further aspect of the invention to provide an *in vitro* use of at least two distinct bacteriophages, at least two distinct nucleic acids, or at least two distinct polypeptides as described herein, as a combined preparation for simultaneous, separate or sequential decontamination of a product contaminated with *E. coli.* It should be understood that preferred combined preparations of at least two (or more) distinct bacteriophages are as described for antibacterial compositions.

### Diagnostic and prognostic methods

In another aspect, the bacteriophages, or components thereof, according to the present invention can be used to predict or determine the efficacy of a bacteriophage therapy in a subject.

Accordingly, a further aspect of the invention resides in a method for predicting or determining the efficacy of a bacteriophage therapy in a subject, comprising determining *in vitro* a lytic activity of one or more bacteriophage, nucleic acid, polypeptide, or antibacterial composition as described herein, to an *E. coli* strain from a sample of said subject, wherein the presence of a lytic activity of said bacteriophage, nucleic acid, polypeptide, or antibacterial composition, to said *E. coli* strain from said sample is indicative of an efficient treatment.

To do so, the skilled practitioner may need to test separately several distinct bacteriophages, nucleic acids, polypeptides, or antibacterial compositions of the present invention in order to identify the one or more than one that is lytic to the particular *E. coli* strain(s).

In another aspect of the present invention, the bacteriophages, or components thereof, as disclosed herein can be used to determine if a patient infected with a particular *E. coli* strain(s) will respond or not to a bacteriophage therapy. Associating a patient's response to such treatment can indeed elucidate new opportunities for treatment in non-responding patients or indicate one treatment over other treatment choices.

In that context, the present invention pertains to a method for selecting a subject or determining whether a subject is susceptible to benefit from a bacteriophage therapy, comprising determining *in vitro* a lytic activity of one or more bacteriophage, nucleic acid, polypeptide, or antibacterial composition as described herein, to an *E. coli* strain from a sample of said subject, wherein the presence of a lytic activity of said bacteriophage, nucleic acid, polypeptide, or antibacterial composition, to said *E. coli* strain from said sample is indicative of a responder subject.

To do so, the skilled practitioner may need to test separately several distinct bacteriophages, nucleic acids, polypeptides, or antibacterial compositions of the present invention in order to identify the one or more than one that is lytic to the particular *E. coli* strain(s).

A responder subject may further be treated with the identified bacteriophage(s), or component(s) thereof, of the invention that is lytic against the *E. coli* strain infecting said subject. Accordingly, in a preferred embodiment, the method further comprises a step of treating said subject by said one or more bacteriophage, nucleic acid, polypeptide, or antibacterial composition having a lytic activity to the *E. coli* strain from said sample.

### EXAMPLES

The present invention will be better understood in the light of the following detailed experiments. Nevertheless, the skilled artisan will appreciate that the present examples are not limitative and that various modifications, substitutions, omissions, and changes may be made without departing from the scope of the invention.

### MATERIALS AND METHODS

### Bacterial strains

Two *E. coli* laboratory strains were used as indicator strains to screen coliphages. MAC1403 is an MG1655-derivative in which the KEIO allele *hsdR:*kanR was added by P1 transduction. Strain C is a natural *E. coli* isolate devoid of restriction activity (referenced under ATCC8739) and its genome is fully sequenced (NC_010468).

954 natural *E. coli* strains were isolated from feces samples of 649 children that were included prospective Copenhagen Prospective Studies on Asthma in Childhood 2010 (COPSAC2010) mother-child cohort (Bisgaard et al., Clinical and experimental allergy: journal of the British Society for Allergy and Clinical Immunology, 2013, 43: 1384-1394). All but 55 strains came from one-year-old children fecal samples, the remaining 55 from two-years-old children fecal samples. Dilutions of the fecal samples were plated and grown aerobically. One to five colonies per sample, with different morphologies, were further cultivated and stored at -80°C with 20% glycerol. Species determination was then determined biochemically, as already described (Stokholm et al. J Allergy Clin Immunol, 2016, 138: 881-889 e882).

Of these natural *E. coli* isolates, at least 70 *E. coli* strains from one-year-old and two years old children were chosen to constitute a sub-panel for interaction analyses ,). For this *E*. *coli* panel, a further classification was achieved using multiplex PCR, to identify the strains to the phylogroups A, B1, B2, C, D, E or cryptic clades (Clermont et al., Microbiol Rep, 2013, 5: 58-65). Briefly, a first quadruplex PCR reaction targeting three genes (*chuaA, yjaA* and *arpA*) and a DNA fragment (TspE4.C2) was carried out with 1 µL of overnight culture. PCR products were then migrated on 2% agarose gel and exposed under UV light to reveal the isolate profile. The quadruplex genotype (presence/absence of the four PCR amplicons) was used to determine the phylogroup of each isolate. A second duplex PCR was done when the first quadruplex PCR was insufficient to discriminate between two phylogroups.

### Detection of temperate coliphages produced by natural E. coli strains

Natural *E. coli* isolates were cultivated in 96-well plates in LB medium. Following an overnight incubation at 37°C, cultures were filtered through a 0.2 µm membrane filter (Pall AcroPrep Advance 96 filter plate). Supernatants were diluted in SM buffer (50 mM Tris pH 7.5, 100 mM NaCl, 10 mM MgSO4) at three different dilutions (10-1, 10-2, 10-3). Next, double-layer plates with each bacterial indicator strain (MAC1403 or C) were inoculated with 5 µl aliquots of serially diluted phages using a Hamilton robotic system. Plates were then incubated overnight at 37°C, and single plaque detection was used as an indicator of phage production. Each natural strain was tested twice on the two indicator strains. For strains where results were unclear, a third test was conducted. Phage concentrations in overnight cultures were estimated from the number of plaque forming units (PFU) detected at a given dilution. Among all phages obtained, about 70 culture supernatants were randomly selected for further study (all of them from one-year-old children samples). Eighteen of them were plaque purified on either one of the two indicator strains, and amplified to produce larger stocks for DNA extraction and genome sequencing.

### Detection of temperate and virulent coliphages in fecal viromes

Coliphages, both virulent and temperate, were collected in the fecal samples of 1-year-old children. Briefly, viromes were fractionated from 0.15 g of feces suspended into 29 ml 1x SM buffer (100 mM NaCl, 8 mM MgSO4•7H2O, 50 mM Tris-Cl with pH 7.5). The suspension was gently centrifuged (5000 x g for 30 min. at 4°C) to pellet larger particles and then filtered through 0.45 µm Minisart® High FlowPES syringe filter (Cat. No. 16533, Sartorius, Germany). The filtrate was concentrated using Centriprep® Ultracel® YM-50K kDa units (Cat. No. 4310, Millipore, USA) until a final volume of 500 µL virome concentrate was obtained. From such viromes, 140µL was taken for DNA extraction, and the rest was stored at 4°C, and filtered through 0.2 µm Supor membrane (Acrodisc 13 mm syringe filter, PALL) prior usage for phage detection. To detect the presence of phages, 10 µl of each of the 649 virome samples were spotted on a double layer plate inoculated with one of the two indicator strains, and incubated at 37°C. The presence of confluent lysis or individual plaques was noted after overnight incubation. Within each 10 µL spot, in most cases there were less than 30 of them. In the few cases where lysis was confluent, dilutions were spotted in order to estimate the coliphage titer of the virome. The first phages isolated were also purified twice by plaque streaking on the same indicator strain, amplified by confluent lysis on plates, and stored in SM buffer at 4°C before being studied in greater detail.

### Coliphage host range determination

Using the collection of temperate coliphages isolated from strains, and coliphages isolated from viromes, it was determined which ones were able to grow on the panel of at least 70 natural *E. coli* strains. In addition, 16 virulent coliphages from the d'Hérelle collection were tested as a reference. To accomplish this, 1 mL of overnight culture was washed over the surface of a squared plate of dried LB supplemented with 5 mM CaCl₂, 10 mM MgSO₄ and 0.2% maltose, and the excess liquid was decanted. Then, 5 µl of filtered supernatants containing coliphages or purified coliphage stocks were applied on plates inoculated with the strain of interest. Plates were incubated overnight at 37°C. The interactions were classified as either positive (with clear or turbid lysis plaques or confluent lysis spots) or negative (no lysis spots and no lysis plaques). The host-range determination was tested twice for each sample containing coliphages and if results were unclear, a third test was conducted.

### Relative abundance of E. coli in samples from 1-year-old children

16S rRNA amplicon sequencing of the microbiota of the children has been performed, as previously described (Stokholm J et al., Nat Commun, 2018, 141: 02573-2). To do so, all operational taxonomic units (OTUs) formerly assigned to *Enterobacteria* were reanalyzed against the Silva database. This allowed adding two abundant *Enterobacteria* to the *E. coli* group. New *E. coli* to total OTU ratios for the 75 samples in which coliphages were found and characterized. In consequence, the overall relative abundance *of E. coli* increased (1% median value).

### Coliphage genome sequencing

For 50 of the coliphages isolated, DNA was extracted from crude phage stocks, as previously described (Lossouarn et al., Viruses, 2019, 11(1)). Briefly, crude lysates were PEG precipitated, followed by a DNaseI/RNase treatment to remove external contamination, and DNA was extracted with the Wizard DNA clean-up system (Promega). The detection of a DNA band for 5 µL of sample on an agarose gel, even if faint (∼ above 2 ng), was considered sufficient to go for sequencing. Sequencing libraries were prepared using the Trueseq nano Sample Preparation kit, and samples were sequenced with an Illumina Miseq run. Read quality trimming was performed using Trimmomatic (Bolger et al., Bioinformatics, 2014, 30:2114-2120) and following options ILLUMINACLIP: Trueseq_nano.txt:2:30:10 LEADING:3 TRAILING:3 SLIDINGWINDOW:4:20 MINLEN:200. Trueseq nano kit tags were sometimes present inside reads, so they were systematically removed using the following sequences for clipping: CTGTCTCTTATACACATCTCCGAGCCCACGAGAC for read 1 of the pair (SEQ ID NO: 16), and TGTAGATCTCGGTGGTCGCCGTATCATTAAA for read 2 (SEQ ID NO: 17). Genome assembly was performed with spades v3.13.0 (Bankevich et al., J Comput Biol, 2012, 19:455-477). using only paired reads, and following options --only-assembler--meta -k 21,33,55,77,99,127. Phage genome was considered complete when spades returned a single contig with high coverage. Repeats at the contig ends were removed prior choosing genome orientation and nt 1 position, relative to reference genomes. In three cases where spades returned more than one contig with high coverage, assembly was completed manually, based on overlapping sequences at contig ends.

### Dotplot analysis, genome annotations and comparisons

Assembled phage genomes were compared to the NCBI Viruses section database using Megablast to choose a well-known reference genome for each genus. Dot plot graphs were generated using Gepard (word size=10) (Krumsiek et al., 2007, Bioinformatics, 23: 1026-1028). Automatic ORF calling and annotation were performed using RAST (Overbeek et al., Nucleic Acids Res, 2014, 42: D206-214) and the Virus genome option. Manual editing was done for the connector genes detected with Virfam, as well as for lambda morons. Reference phage phAPEC8 being poorly annotated, its annotation was completed using distant homology searches, thanks to Phagonaute interface (Delattre et al., 2016, Virology, 496: 42-50). The T5s and Mu automatic annotations were completed following DT57C and Mu genbank files. tRNA were searched with tRNAscanSE 2.0 (Lowe et al, 2016, Nucleic Acids Res, 44: W54-57). A systematic search for reverse transcriptases, frequently found in intestinal phages (Cornuault, et al., Microbiome, 2018, 6(1): 65), was conducted with hmmscan against Pfam profile PF00078, and none was found. Antibiotic resistance genes were searched using ResFam core profiles and hmmscan as indicated by Tobe et al., Proc Natl Acad Sci U S A, 2006, 103: 14941-14946. All virulence factors detected thanks to the Virulence Factor Database had been correctly assigned by RAST, except for the *espM2* and *espV* genes.

### RESULTS

### Overview of the study

To study intestinal *E. coli* phage-host interactions, phages from an unselected prospective cohort of 1-year-old children were isolated, based on 649 fecal samples. The investigations were carried out are summarized on Figure 1. In a first investigation, the temperate phages hosted in 954 *E. coli* strains previously isolated from these same fecal samples were analyzed (0 to 5 isolates per sample). These *E. coli* strains were searched for the presence of virions spontaneously induced in *in vitro* culture supernatants by plaquing them on two indicator strains. Next, it was aimed to collect coliphages, both virulent and temperate, present in the fecal samples. To do so, viromes were fractionated from 0.15 g of feces, using a gentle method based on fecal sample dilution, filtration, and concentration through ultrafiltration. From such viromes, plaque isolation was then performed, on the same two indicator strains used for strain supernatants. It was also investigated if these phages could lyse *E. coli* strains that were isolated from the same, or other samples from the same cohort. In total, over a hundred of phages were isolated (about 70 from *in vitro E. coli* culture supernatants and about 70 from viromes) and tested for their capacity to grow on a panel of at least 70 *E. coli* isolates that were representative of the community present in fecal samples of 1-year-old children. Lastly, the genomes of 50 phages (18 isolated from strain supernatants, as well as 17 temperate and 15 virulent from the viromes of fecal samples) were sequenced and analyzed at the molecular level.

The gist of the present invention lies in the identification and characterization of 15 new virulent coliphages isolated in the viromes of infant fecal samples, which are highly lytic against a broad range *of E. coli* strains.

### A majority of the E. coli strains from 1-year-old children are lysogens producing virions spontaneously in their supernatants

Two laboratory *E. coli* strains were chosen for phage isolation based on their high phage sensitivity. Strain MAC1403 (MG1655 hsdR::kanR) is a K12 derivative (phylogroup A) devoid of active prophage and with no DNA restriction activity. It has a lipopolysaccharide (LPS) with a K12-type outer-core component, and is devoid of O-glycosylation (rough phenotype). Strain C also belongs to phylogroup A, is restrictionless, has an LPS devoid of O-glycosylation but has an R1 type outer-core. The *E. coli* C strain has long been recognized as an efficient indicator strain.

For the 954 natural *E. coli* isolates from the fecal samples of 1-year-old children, filtered supernatants of 96-well plate cultures were serially diluted and plated on the two indicator strains. The observation of isolated plaques was considered proof of the presence of temperate phage(s) in the supernatant (Figure 5). The detection limit was 10² pfu/mL of supernatant. Remarkably, 63.4% of the strains produced virions that were able to form plaques on either or both of our indicator strains. The C strain was clearly a better host for phage growth, with 57.5% of the supernatants producing plaques, compared to only 35.6% on the MG1655 derivative. However, the replication of some of the phages was limited to only one of the two strains, confirming the importance of using these two host strains. In all cases, confluent lysis spots and isolated plaques exhibited a turbidity zone typical of temperate phages (see two examples, Figure 1B, left panels). Supernatant titers ranged between 10² and >10⁶ pfu/mL (Figure 5C). The remaining 37% of the strains did not produce virions spontaneously in their supernatant under the tested laboratory conditions. Thus, at least 63% of *E. coli* isolates from the infant gut are lysogens. This is likely an underestimate since only virions produced spontaneously in the *in vitro* culture supernatant and growing as plaques on either of the two indicator strains were detected. Among the phages isolated, about 70 were selected for further analysis. These phages were chosen based on their different plaque characteristics (size, turbidity), with a maximum of one phage per initial fecal sample.

### Isolation of temperate and virulent phages from the intestinal viromes of 1-year-old children

Coliphages were isolated from viromes by direct spotting (10 µL) on lawns of the two indicator strains, without an enrichment step. Since *E. coli* is a dominant species in the microbiota, its phages should be easily isolated. Among the virome samples, 23.7% were found to contain coliphages (>500 pfu/gr of feces) infecting at least one of the two indicator strains. Within each 10 µL spot, all plaques were of similar size and phenotype. Children who had coliphages isolated had a significantly higher proportion of *E. coli* OTUs compared to those with coliphage negative samples (Mann-Whitney two-tailed test, P<0.0001, Figure 5D). Interestingly, some of these phages produced clear plaques, suggesting that virulent phages had been isolated (Figure 5B, right side). Among the first ≃70 isolated plaques, which were studied further, 27% were virulent. The virulent phages were obtained from fecal samples that presented modified population parameters compared to temperate phages. Firstly, they contained slightly higher *E. coli* OTU proportions (Figure 2A). Secondly, the phage titer itself was significantly higher as well (median value 3.1x10⁴ PFU/gr of feces for virulent, compared to 2.8x10³ for temperate phages, Figure 2B).

Taken altogether, the viromes of healthy children contain both temperate and virulent coliphages, which were further purified and analyzed in greater detail.

### Contrasted host ranges between temperate and virulent coliphages

Next, the capacity of the selected phages (about 70 spontaneously induced and about 70 isolated from viromes) to replicate was tested on a panel of at least 70 *E. coli* strains. A maximum of one strain per child was chosen, half of which were proven lysogens (*i.e*. strains for which phages could be detected in supernatants). It was ensured that none of the selected *E. coli* strains originated from a child from which phages to be tested had been isolated (this was also tested, later, see below). A PCR typing was performed on the tested *E. coli* strains in order to determine their phylogroup (Clermont et al., Environ Microbiol Rep, 2013, 5:58-65). A clear dominance of the B2 type (41.3% of strains), and a paucity of the A type (5%) were observed. Clades E, B1 and C had intermediate levels (11-19%, Table 4). Interestingly, the B2 clade was significantly enriched in lysogens, while B1 and A were significantly depleted in lysogens (Figure 7A).

**Table 4. Proportion of each phylogroup within the natural E. coli strains used for the interaction study.**

| *E. coli* strains features | | % |
|---|---|---|
| containing active prophages | | 57 |
| not phage producer | | 43 |
| Phylotype | A | 5.3 |
| | B1 | 13.3 |
| | B2 | 41.3 |
| | C | 10.7 |
| | D | 8 |
| | E | 18.7 |
| | E. clades | 2.7 |

The temperate phages displayed a remarkably low level of infectivity (Figure 3A). At most, 10% of the strains were lysed by a temperate phage. Likewise, among the panel of the phages isolated from viromes and purified on either one of the two indicator strains against the panel of *E. coli* isolates, a low level of infectivity was observed, with at most 10% of the strains lysed (Figure 3B). In stark contrast, 15 out of the isolated virulent phages lysed between 12% and 73% of the strains (Figure 3C and Table 5 below). Interestingly, four virulent phages lysed at least 60% of all strains tested (three rV5 and one phAPEC8, see Table 5 below). Lastly, 16 known virulent coliphages from the Felix d'Hérelle Reference Center for Bacterial Viruses were used as a reference set, and tested against the same panel of *E. coli* strains (Figure 3D). Their infectivity fell between the temperate and the virulent phage sets, as between 0% and 40% of the strains were lysed by this reference phage set.

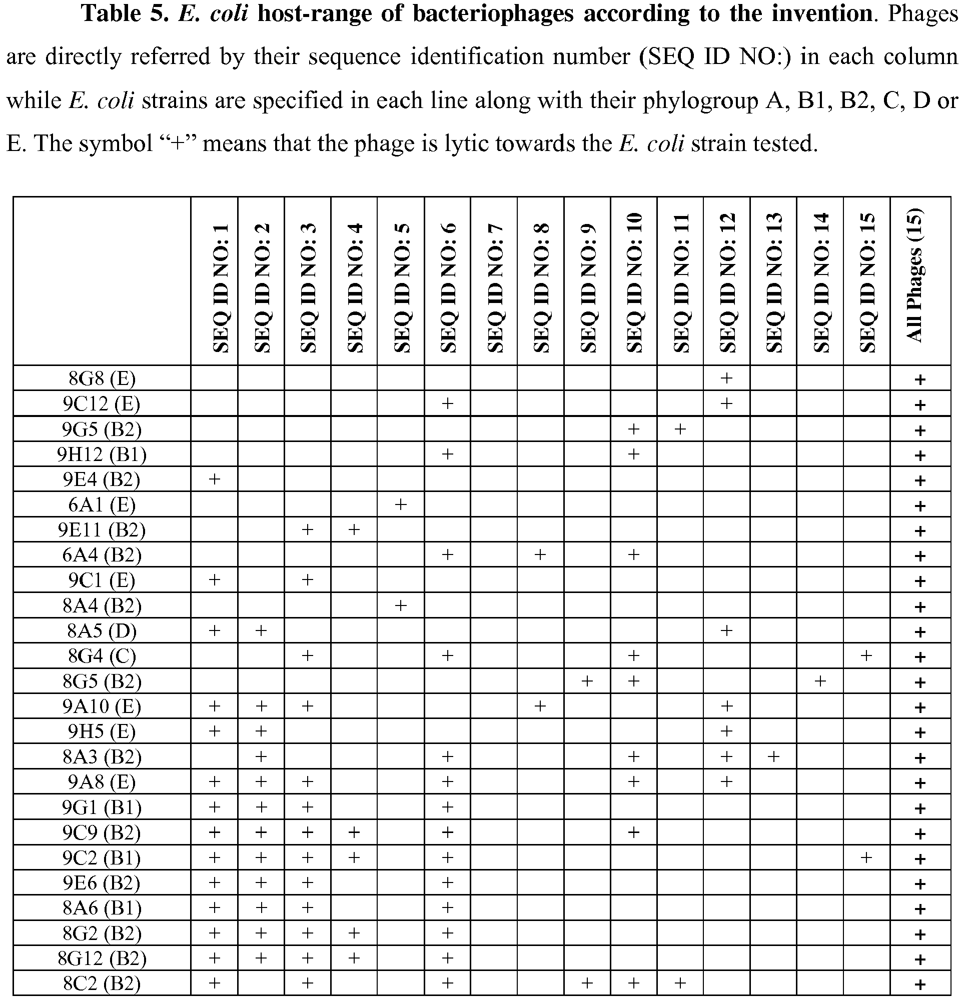

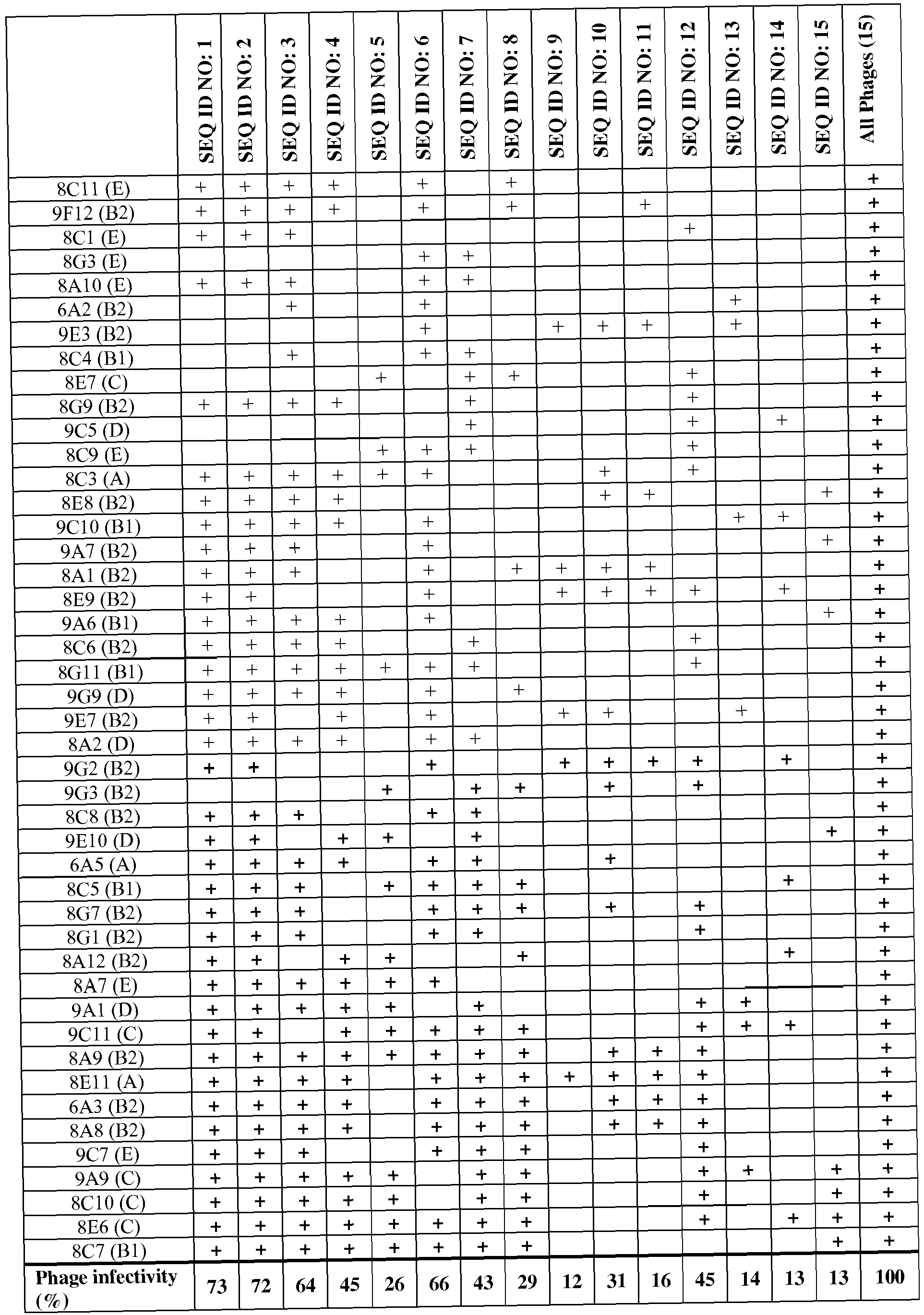

These results highlight that the overall infectivity of these three phage groups (temperate, virulent, and reference virulent) are clearly distinct (Figure 7B). The virulent coliphages isolated from the infant gut were the most infectious towards the strains of this niche, followed by the reference virulent phages, and then the temperate phages. The variations in phage sensitivity could not be related to the lysogeny status of the tested strains, as lysogens and non-lysogens were equally resistant to phages (Figure 7C). The *E. coli* phylogroups also behaved similarly (Figure 7D).

To complement the present analysis, each phage was tested on a few *E. coli* strains isolated from the same fecal sample (Figure 7D). The overall trend observed was conserved. All but 3 of the 130 temperate phages were unable to replicate on the strains of their ancestral ecosystem, whereas 9 of the 20 virulent phages replicated on at least one strain of the niche from which it was initially isolated.

### Characteristics of the sequenced virulent coliphages genomes

The virulent coliphages were of most interest in the present study, because of their capacity to lyse a wide range of *E. coli* strains. Their characteristics are summarized in Table 6 below, which indicates the phage name attributed, their SEQ ID reference number, their infectivity towards the *E. coli* strains tested, their VIRFAM classification, as well as the identification of auxiliary metabolic genes (AMG).

**Table 6. Characteristics of the sequenced virulent coliphages genomes (source: virome)**

| **Phage name** | **SEQ ID NO:** | **Genome length (bp)** | ***E.coli* infectivity (%)** | **Virfam classification** | **AMG** |
|---|---|---|---|---|---|
| **rV5_ev156** | 1 | 136 385 | 73 | M 1,7 | HPF |
| **rV5_ev147** | 2 | 136 384 | 72 | M 1,7 | HPF |
| **rV5_ev158** | 3 | 138 177 | 64 | M 1,7 | HPF |
| **rV5_ev146** | 4 | 131 919 | 45 | M 1,7 | HPF |
| **rV5_ev168** | 5 | 138 171 | 26 | M 1,7 | HPF |
| **phAPEC8_ev052** | 6 | 150 152 | 66 | M 1,7 | RmlA, RmlB |
| **T5_ev212** | 7 | 108 546 | 43 | S 1,4 | DHFR |
| **T5_ev219** | 8 | 110 393 | 29 | S 1,4 | DHFR |
| **VpaE1_ev035** | 9 | 88 002 | 12 | M 1,7 | DHFR |
| **VpaE1_ev108** | 10 | 87 749 | 31 | M 1,7 | DHFR, Prs |
| **VpaE1_ev078** | 11 | 87 688 | 16 | M 1,7 | DHFR, Prs |
| **T4_ev151** | 12 | 167 097 | 45 | M2 | DHFR, Prs |
| **T4_ev240** | 13 | 166 954 | 14 | M2 | DHFR, Prs |
| **Stevie_ev116** | 14 | 48 646 | 13 | S 1 | n/a |
| **Gluttony_ev152** | 15 | 44 825 | 13 | S 1,5 | n/a |

| | | | | | |
|---|---|---|---|---|---|
| (M: myophage, S: siphophage. The number next to M or S indicates the VIRFAM classification number (Lopes et al, BMC Genomics, 2014, 15: 1027). Type 1 is further sub-divided into 10 clusters, which number is given next to the Type 1 number; for example, M 1,7 stands for myophage Type 1 cluster 7. AMG: auxiliary metabolic gene; HPF: hibernation promoting factor, DHFR: Dihydrofolate reductase, Prs: Ribose-phosphate pyrophosphokinase; RmlA: glucose-1-phosphate thymidylyltransferase; RmlB: Glucose-1-phosphate thymidylyltransferase, n/a: no data available). | | | | | |

Among the 15 sequenced genomes from virulent phages, five were closely related to the myophage rV5 of the *Vequintavirinae* subfamily, and one of them infected up to 73% of the tested *E. coli* strains, namely rV5_ev156 (range 26%-73%, average 56%). In addition, a phAPEC8-like phage was isolated, which is distantly related to rV539 (18% shared proteins), that infected 66% of all strains tested (phAPEC8_ev052). This underlines the remarkable capacity of members of the *Vequintavirinae* subfamily to adapt and infect various *E. coli* strains isolated from the infant gut.

The next most infectious phages were related to the siphophage T5 (two isolates, named herein T5_ev212 and T5_ev219) and myophage T4 (two isolates, named herein T4_ev151 and T4_ev240). Of note, the host range was distinct within each phage pair. The two T5-like phages infected 29% and 43% of the strains, while the two T4-like phages infected 14% and 45% of the strains, respectively.

A set of three myophages were homologous to VpaE1 (88,403 bp), a member of the Felixo1virus genus, which can replicate over a wide range of temperatures (from 9 to 45°C) and has interesting host range properties (Simoliunas et al., Viruses, 2015, 834(7): 6163-6181). The phages are described as in above Table 6 as VpaE1_ev035, VpaE1_ev108 and VpaE1_ev078. One of the particularities of the phage VpaE1 is that its receptor is composed of a truncated core LPS. It was suggested that phage VpaE1 preferentially targets *E. coli* mutants whose LPS cores are truncated to resist infection by other phages such as T4. However, a close relative of VpaE1, called Alf5, was previously isolated and infects strain K12, which has a complete core LPS (Alijosius et al., Genome Announc, 2017, 5: 837). Phage Alf5 encodes 3 different alleles of the tail fiber proteins involved in host recognition. A comparison of the newly isolated VpaE1 phages to these VpaE1 and Afl5 showed VpaE1_ev035, VpaE1_ev108 and VpaE1_ev078 had a tail fiber more closely related to VpaE1_gp74 than to its counterpart in Af15. This may explain the limited host range by the VpaE1-like phages isolated in the present study (12%, 16% and 31% of the tested strains).

Finally, the two remaining virulent phages had smaller genome sizes and were the ones that infected fewer strains. These were, respectively, homologous to Stevie (49,816 bp, referred herein as Stevie_ev116) and Gluttony (44,513 bp, referred herein as Gluttony_ev152), and infected 13% of the strains. Gluttony is an *E. coli* bacteriophage isolated from the microbiota of a female bladder. Its neck module classification with Virfam places Gluttony not far from PA73, in type 1, cluster 5. Stevie on the other hand is related to T1 and was originally isolated from *Citrobacter* (Shaw et al., Genome Announc, 2015, 3:1).

Of note, various auxiliary metabolic genes (AMG) were identified in the virulent genes (see above Table 6 and Figure 4A), while antibiotic resistance genes and virulence factors (at least those known) were not detected. Most of the AMG detected in these phages were related to nucleotide metabolism. The five rV5-like phages encoded a ribosome hibernation factor (*hpf* gene) as observed in myophage rV5 (Mizuno et al., Nat Commun, 2019, 10: 752).

Confronting the interaction matrix with genomic data revealed that closely related phages had different host ranges. In particular, a marked gradation of strain infectivity was observed for reference phage T5 (8% of strains infected) compared to T5_ev219 (29% of strains infected) and T5_ev212 (43% of strains infected). Two encoded receptor-binding proteins have been reported in phage T5, allowing two different bacterial receptors to be recognized: FhuA and BtuB (Golomidova et al., Viruses, 2016, 8: 879). Moreover, T5 encodes various types of L shaped tail fibers, helping to overcome the bacterial surface shielding by O-377 glycosylated sugars. A comparison of the genome of two new T5-like phages with reference phages T5 and DT57C showed good overall synteny, except at two loci. One locus was at the receptor binding protein, which was most similar to that of phage DT57C (73% amino acid identity) binding the BtuB bacterial 380 receptor. The other clear interruption of synteny occurred at the position of the gene for the L-shape 381 tail fiber proteins, which was different in each genome (Figure 4B). This may explain the different host 382 ranges (Figure 4B right part).

### CONCLUSION

The present study is the first of its kind to investigate coliphage growth in *E. coli* strains isolated from children.

Overall, the 15 virulent phages isolated from the infant gut virome were far more diverse than the temperate phages identified, with some exhibiting not only remarkably high infective powers but also contrasted host range between each other.

Most strikingly, these virulent phages not only have a much greater infectivity than the temperate phages but also display the broadest host-range in *E. coli* strains, and, as such, can be used *in vitro* or *in vivo,* individually or in combination, to kill a wide spectrum of *E. coli* strains.

## Claims

1. A bacteriophage having lytic activity to an *Escherichia coli* strain and having a genome comprising a nucleotide sequence selected from any one of SEQ ID NO: 1 to 15 or a nucleotide sequence having at least 97% identity thereto.

2. The bacteriophage according to claim 1, which is any one of:
- a myophage having a genome sequence comprising a nucleotide sequence selected from any one of SEQ ID NO: 1 to 6 and 9 to 13, or a nucleotide sequence having at least 97% identity thereto; or
- a siphophage having a genome sequence comprising a nucleotide sequence selected from any one of SEQ ID NO: 7 to 8 and 14 to 15, or a nucleotide sequence having at least 97% identity thereto.

3. The bacteriophage according to claim 1 or 2, which is any one of:
- a rV5-like bacteriophage having a genome sequence comprising a nucleotide sequence selected from any one of SEQ ID NO: 1 to 5, or a nucleotide sequence having at least 97% identity thereto;
- a phAPEC8-like bacteriophage having a genome sequence comprising the nucleotide sequence SEQ ID NO: 6, or a nucleotide sequence having at least 97% identity thereto;
- a T5-like bacteriophage having a genome sequence comprising the nucleotide sequence SEQ ID NO: 7 or 8, or a nucleotide sequence having at least 97% identity thereto;
- a VpaE1-like bacteriophage having a genome sequence comprising a nucleotide sequence selected from any one of SEQ ID NO: 9 to 11, or a nucleotide sequence having at least 97% identity thereto;
- a T4-like bacteriophage having a genome sequence comprising the nucleotide sequence SEQ ID NO: 12 or 13, or a nucleotide sequence having at least 97% identity thereto;
- a Stevie-like bacteriophage having a genome sequence comprising the nucleotide sequence SEQ ID NO: 14, or a nucleotide sequence having at least 97% identity thereto; or
- a Gluttony-like bacteriophage having a genome sequence comprising the nucleotide sequence SEQ ID NO: 15, or a nucleotide sequence having at least 97% identity thereto.

4. The bacteriophage according to any one of the preceding claims, which has lytic activity to an *Escherichia coli* gut strain, and/or to a pathogenic and/or drug-resistant *Escherichia coli* strain.

5. An isolated nucleic acid comprising a nucleotide sequence selected from any one of SEQ ID NO: 1 to 15 or a nucleotide sequence having at least 97% identity thereto.

6. An isolated polypeptide encoded by a bacteriophage as defined in any one of claims 1 to 4 or by a nucleic acid as defined in claim 5.

7. An antibacterial composition comprising at least two distinct bacteriophages having lytic activity to an *Escherichia coli* strain, said at least two distinct bacteriophages being selected from the bacteriophages having a genome comprising a nucleotide sequence selected from any one of SEQ ID NO: 1 to 15 or a sequence having at least 90% identity thereto.

8. The composition according to claim 7, wherein said at least two distinct bacteriophages are selected from the group consisting of:
- a myophage having a genome sequence comprising a nucleotide sequence selected from any one of SEQ ID NO: 1 to 6 and 9 to 13, or a nucleotide sequence having at least 90% identity thereto; and
- a siphophage having a genome sequence comprising a nucleotide sequence selected from any one of SEQ ID NO: 7 to 8 and 14 to 15, or a nucleotide sequence having at least 90% identity thereto.

9. The composition according to claim 7 or 8, wherein said at least two distinct bacteriophages are selected from the group consisting of:
- a rV5-like bacteriophage having a genome sequence comprising a nucleotide sequence selected from any one of SEQ ID NO: 1 to 5, or a nucleotide sequence having at least 90% identity thereto;
- a phAPEC8-like bacteriophage having a genome sequence comprising the nucleotide sequence SEQ ID NO: 6, or a nucleotide sequence having at least 90% identity thereto;
- a T5-like bacteriophage having a genome sequence comprising the nucleotide sequence SEQ ID NO: 7 or 8, or a nucleotide sequence having at least 90% identity thereto;
- a VpaE1-like bacteriophage having a genome sequence comprising a nucleotide sequence selected from any one of SEQ ID NO: 9 to 11, or a nucleotide sequence having at least 90% identity thereto;
- a T4-like bacteriophage having a genome sequence comprising the nucleotide sequence SEQ ID NO: 12 or 13, or a nucleotide sequence having at least 90% identity thereto;
- a Stevie-like bacteriophage having a genome sequence comprising the nucleotide sequence SEQ ID NO: 14, or a nucleotide sequence having at least 90% identity thereto; and/or
- a Gluttony-like bacteriophage having a genome sequence comprising the nucleotide sequence SEQ ID NO: 15, or a nucleotide sequence having at least 90% identity thereto.

10. A bacteriophage as defined in any one of claims 1 to 4, or a nucleic acid as defined in claim 5, or a polypeptide as defined in claim 6, or an antibacterial composition as defined in any one of claims 7 to 9, for use as a medicament.

11. The bacteriophage, or nucleic acid, or polypeptide, or antibacterial composition for use according to claim 10, in the treatment of an *E. coli* infection in a subject or for restoring the microbial flora in a subject.

12. At least two distinct bacteriophages as defined in any one of claims 1 to 4, or at least two distinct nucleic acids as defined in claim 5, or at least two distinct polypeptides as defined in claim 6, as a combined preparation for simultaneous, separate or sequential use in therapy.

13. *In vitro* use of a bacteriophage as defined in any one of claims 1 to 4, or a nucleic acid as defined in claim 5, or a polypeptide as defined in claim 6, or an antibacterial composition as defined in any one of claims 7 to 9, for decontamination of a product contaminated with *E. coli.*

14. A method for predicting or determining the efficacy of a bacteriophage therapy in a subject, wherein the method comprises the step of determining *in vitro* a lytic activity of one or more bacteriophage as defined in any one of claims 1 to 4, or nucleic acid as defined in claim 5, or polypeptide as defined in claim 6, or antibacterial composition as defined in any one of claims 7 to 9, to an *E. coli* strain from a sample of said subject, the presence of a lytic activity of said bacteriophage, nucleic acid, polypeptide, or antibacterial composition to said *E.coli* strain from said sample being indicative of an efficient treatment.

15. A method for selecting a subject or determining whether a subject is susceptible to benefit from a bacteriophage therapy, wherein the method comprises the step of determining *in vitro* a lytic activity of one or more bacteriophage as defined in any one of claims 1 to 4, or nucleic acid as defined in claim 5, or polypeptide as defined in claim 6, or antibacterial composition as defined in any one of claims 7 to 9, to an *E. coli* strain from a sample of said subject, the presence of a lytic activity of said bacteriophage, nucleic acid, polypeptide, or antibacterial composition to said *E.coli* strain being indicative of a responder subject.
